# EUROPEAN PATENT APPLICATION

(11) **EP 1 987 834 A2**
(43) Date of publication of application: **05.11.2008**
(21) Application number: 08101433.4
(22) Date of filing: 08.02.2008
(51) Int. Cl.: A61K 31/5375, A61P 25/28

(54) **Substituted piperidines as therapeutic compounds**

(30) Priority: 13.02.2007 EP 07102214
(71) Applicant: Speedel Experimenta AG, 4123 Allschwil (CH)
(72) Inventor: Herold, Peter, 4123, Allschwil (CH); Mah, Robert, 4123, Allschwil (CH); Tschinke, Vincenzo, 4123, Allschwil (CH); Behnke, Dirk, 4123, Allschwil (CH); Marti, Christiane, 4123, Allschwil (CH); Jotterand, Nathalie, 4123, Allschwil (CH); Stutz, Stefan, 4123, Allschwil (CH)
(74) Representative: Maué, Paul Georg

(57) **Abstract**

Use of compounds of the general formula (1) 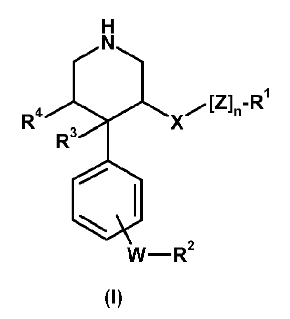 and pharmaceutically acceptable salt thereof, in which R¹, R², R³, R⁴, W, X, Z and n have the definitions illustrated in detail in the description, as beta-secretase, cathepsin D, plasmepsin II and/or HIV protease inhibitors.

## Description

### Field of the Invention

The present invention relates to the use of substituted piperidines as beta-secretase-, cathepsin D-, plasmepsin 11- and/or HIV-protease-inhibitors.

### Background of the Invention

With regard to beta-secretase-, cathepsin D-, plasmepsin 11- and/or HIV-protease-inhibition, there is still a need for highly potent active ingredients. In this context, the improvement of the pharmacokinetic properties is at the forefront. These properties directed towards better bioavailability are, for example, absorption, metabolic stability, solubility or lipophilicity.

### Alzheimer Disease aspartyl protease: Beta-Secretase

Alzheimer's disease (AD) is a progressive degenerative disease of the brain. The symptoms of AD include progressive memory loss, language difficulty and ultimately loss of basic neural function and death. The biomarkers in the central nervous system for AD include amyloid plaques, intracellular neurofibrillary tangles and activated microglia. The appearance of these three markers is likely to contribute to the neuronal cell death and memory loss observed in AD.

Beta-amyloid is a defining feature of AD and now believed to be a causative precursor in the development of the disease. Amyloidogenic plaques and vascular amyloid angiopathy also characterize the brains of individuals with Trisomy 21 (Down's Syndrome), Hereditary Cerebral Hemorrhage with Amloidosis of the Dutch-Type (HCHWA-D) and other neurodegenerative disorders.

Beta-amyloid plaques are predominantly composed of amyloid beta peptide (A-beta, also sometimes designated betaA4). The A-beta peptide is derived by proteolysis of the beta amyloid precursor protein (APP). Beta-APP is processed by three distinct ordered enzymatic activities. The bulk of beta-APP is processed via alpha-secretase in a non-amyloidogenic pathway. A small fraction of beta-APP is cleaved by beta-secretase activity to generate the membrane-bound C-terminal fragment C99. Gamma-secretase cleaves C99 to generate the amyloidogenic A-beta peptide of 39-42 amino acids. The aspartyl protease activity of beta-secretase has been disclosed using varied nomenclature, including BACE (beta-site APP cleaving enzyme), Asp and memapsin.

The significance of beta-secretase cleavage of beta-APP as a critical step in the generation of AD is underscored by the observation that human mutations at the beta-secretase cleavage subsites (Swedish mutations) of beta-APP lead to increased A-beta production and early onset familial AD. Furthermore, BACE1- knockout mice fail to produce A-beta peptide and present a normal phenotype. When crossed with transgenic mice that overexpress APP, the progeny show reduced amounts of A-beta in brain extracts as compared with control animals. This evidence supports the proposal that inhibition of beta-secretase activity and reduction of A-beta peptide deposits in the brain provides a therapeutic strategy for the treatment of AD and other beta amyloid disorders as described by Verdile et al. (2004) in Pharmacol. Res 50, 397-409.

Compounds that are effective inhibitors of beta-secretase may inhibit beta-secretase-mediated cleavage of APP and the production of A-beta peptide. The pharmacological inhibition of A-beta peptide generation may reduce amyloid beta deposits, respectively the formation of plaques. Beta-secretase inhibiting compounds as discussed by Thompson et al. (2005) in Curr. Pharm. Des. 11, 3383-3404 are therefore useful to treat or to prevent diseases that are characterized by amyloid beta deposits or plaques such as AD.

The present invention also relates to methods of treating subjects who have, or in preventing subjects from developing a disease or condition selected from the group consisting of AD, for helping prevent or delay the onset of AD, for helping to slow the proression of AD, for treating subjects with mild cognitive impairment (MCI) and preventing or delaying the onset of AD in those who could progress form MCI to AD, for treating Down's syndrome, for treating humans who have HCHWAD, for treating cerebral amyloid angiopathy, and for treating degenerative dementias

### Alzheimer's Disease aspartyl protease: Cathepsin D

Human cathepsin D is an intracellular aspartic peptidase found mainly in lysosomes. It has a number of housekeeping functions, including the degradation of cellular and phagocytosed proteins. The enzymes may be involved in a variety of disease states, including cancer and Alzheimer's disease (AD). Clinical studies have shown that cathepsin D is overexpressed in breast cancer cells and this seems to be associated with an increased risk for metastasis due to enhanced cell growth. Cathepisn D is also thought to be involved in formation of the beta-amyloid peptide in AD. Recently, several genetic association studies linked cathepsin D with amyloid pathology and Alzheimer's disease as described for example by Davidson et al., (2006) in J. Neurol. Neurosurg. Psychiatry 77, 515-517. The availability of selective and potent inhibitors will help to further define the role of cathepsin D in disease and possibly lead to therapeutic agents.

### Malaria Aspartyl Protease: Plasmepsin I and II

Malaria is considered as one of the most serious infectious diseases in the world, affecting approximately 500 million people. The disease is spread by the anopheles mosquito that is mostly found in tropical regions. The species plasmodium falciparum is responsible for more than 95% of malaria-related morbidity and mortality. Increasingly, plasmodium falciparum is becoming resistant to existing therapies such as chloroquine, mefloquine and sulfadoxime/ pyrimethamine. Thus there is an urgent need for new treatments.

In the erythrocytic stage of the parasite's life cycle the parasite invades the red blood cells of its host consuming up to 80% of the hemoglobin as a source of nutrients for growth and development. Hemoglobin degradation takes place in an acidic vacuole of the parasite and many of the current antimalarial drugs appear to disrupt important vacuolar functions. The food vacuole contains aspartic, cysteine and metallo-proteases, which are all considered to play a role in the process of hemoglobin degradation. At least 10 genes encoding aspartic proteases have been identified in the plasmodium genome. Four of the aspartic proteases have been localized in the acidic food vacuole of the parasite, namely plasmepsin I, II, IV and HAP, a histo-aspartic protease. Inhibitors of plasmepsin I and II have shown efficacy in cell and animal models of malaria, indicating that these enzymes may represent targets for drug discovery as described for example by Coombs et al. (2001) Trends Parasitol 17, 532-537. Indeed, a non-selective inhibitor of aspartic proteases, pepstatin, inhibits the growth of plasmodium falciparum in vitro. Similar results have been obtained with analogs of pepstatin or with immunodeficiency virus protease inhibitors indicating that inhibition of aspartic proteases interferes with the life cycle of plasmodium falciparum as noted for example by Andrews et al. (2006) in Antimicrob. Agents Chemother 50, 639-648.

The present invention relates to the identification of low molecular weight, non-peptidic inhibitors of the plasmodium falciparum protease plasmepsin II or other related aspartic proteases to treat and/or to prevent malaria.

### HIV aspartyl protease: HIV-1 peptidase

First reported in 1981 in a small number of patients, Acquired immunodeficiency syndrome (AIDS) has now become a major epidemic with more than 38 million people infected worldwide, including approximately 1 million in the United States, 580,000 in Western Europe and more than 25 million in Sub-Saharan Africa (http://www.unaids.org). Since AIDS was first clinically identified, scientific and therapeutic progress has been extraordinary. However, AIDS remains out of control, especially in developing countries.

The prognosis of AIDS patients who have full access to current therapies has completely changed since the first cases of AIDS were reported. Today, the median survival for HIV-positive patients receiving treatment exceeds 8 years. The life expectancy for AI DS patients was less than 1 year before AZT was introduced in 1987. This dramatic change is due to the development of effective therapies, to early detection of HIV-positive individuals, and to a sustained effort to analyze and understand viral-resistance mechanisms, which can be overcome by rational drug development and combination therapy.

FDA-approved therapies target three steps of the HIV life cycle: reverse transcription, proteolytic maturation and fusion. Triple therapy, commonly referred to as HIGHLY ACTIVE ANTIRETROVIRAL THERAPY (HAART), is now the standard for treatment. It consists of a protease inhibitor or a non-nucleoside reverse transcriptase inhibitor in combination with two nucleoside reverse transcriptase inhibitors.

Translation of human immunodeficiency virus type-1 (HIV-1) genomic RNA results in the production of two polyprotein precursors, Gag and Gag-Pol. The 55-kDa Gag precursor contains the structural proteins and the 160-kDa Gag-Pol polyprotein contains the functional viral enzymes protease, reverse transcriptase, and integrase. Gag and Gag-Pol polyproteins are transported to the plasma membrane where assembly of type-C retroviruses and lentiviruses typically occurs. During particle assembly, the viral protease cleaves the Gag and Gag-Pol precursors into the structural and functional proteins required for viral replication. The protease activity within the cytoplasma of infected cells allows for the formation of virions which can be released from the cell in the last stages of budding.

The mature HIV-1 protease is an obligatory dimer of identical 11-kDa subunits, each contributing one of the two catalytic aspartic residues. In contrast, the cell-derived members of the aspartic protease family are monomeric enzymes with two Asp-Thr-Gly-containing domains. The unique dimeric structure of the retroviral protease is mainly stabilized by an antiparallel beta-sheet formed by the interdigitation of the amino- and carboxyl-terminal beta-strands of each monomer.

The activation of HIV-1 protease i.e. the dimerization and autocatalytic release from Gag-Pol, is a critical step in the viral life cycle. Inhibition of protease activation causes a severe defect in Gag polyprotein processing and a complete loss of viral infectivity.

As such, the viral protease has become a target for HIV therapeutics, resulting in many HIV protease inhibitors reaching clinical trials as reviewed by Rana et al. (1999) in Pharmacotherapy 19, 35-59 and Morse et al., (2006) in Lancet Infect. Dis. 6, 215-225. Most of these drugs are substrate-based inhibitors, whose design has been facilitated by an abundance of crystal structure data for both the native enzyme and enzyme-inhibitor complexes. Additionally, there are now extensive biochemical data detailing both the catalytic mechanism and the molecular basis for substrate selection.

### Detailed Description of the Invention

Firstly, the present invention relates to the use as beta-secretase-, cathepsin D-, plasmepsin II- and/or HIV-protease-inhibitors of compounds of the general formula 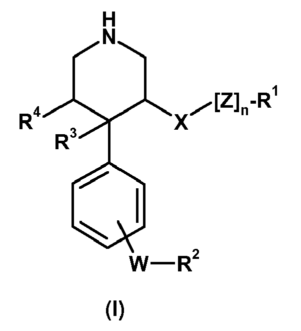 in which
(A) R¹ is aryl when R² is tetrazolyl or imidazolyl, each of which may be substituted by C₁₋₆alkoxy-C₁₋₆alkoxy-C₁₋₆alkyl, C₁₋₆alkoxy-C₁₋₆alkyl, aryloxy-C₁₋₆alkyl, heterocyclyloxy-C₁₋₆alkyl; or
(B) R¹ is aryl when X is -O-CHR⁵-CO-NR⁶-; or
(C) R¹ is aryl when Z is -alk-NR⁶-, where alk is C₁₋₆alkylene, and n is 1; or
(D) R¹ is aryl which is substituted by 1-4 acetamidinyl-C₁₋₆alkyl, acyl-C₁₋₆alkoxy-C₁₋₆alkyl, (N-acyl)-C₁₋₆alkoxy-C₁₋₆alkylamino, C₁₋₆alkoxy, C₁₋₆alkoxy-C₁₋₆alkoxy, C₁₋₆alkoxy-C₁₋₆alkoxy-C₁₋₆alkyl, C₁₋₆alkoxy-C₁₋₆alkyl, (N-C₁₋₆alkoxy)-C₁₋₆alkylaminocarbonyl-C₁₋₆alkoxy, (N-C₁₋₆alkoxy)-C₁₋₆alkylaminocarbonyl-C₁₋₆alkyl, C₁₋₆alkoxy-C₁₋₆alkylcarbamoyl, C₁₋₆alkoxy-C₁₋₆alkylcarbonyl, C₁₋₆alkoxy-C₁₋₆alkylcarbonylamino, 1-C₁₋₆alkoxy-C₁₋₆alkylimidazoi-2-yl, 2-C₁₋₆alkoxy-C₁₋₆alkyl-4-oxoimidazol-1-yl, 1-C₁₋₆alkoxy-C₁₋₆alkyltetrazol-5-yl, 5-C₁₋₆alkoxy-C₁₋₆alkyltetrazol-1-yl, 6-alkoxyamino-carbonyl-C₁₋₆alkoxy, C₁₋₆alkoxyaminocarbonyl-C₁₋₆alkyl, C₁₋₆alkoxycarbonyl, C₁₋₆alkoxycarbonyl-C₁₋₆alkoxy, C₁₋₆alkoxycarbonyl-C₁₋₆alkyl, C₁₋₆alkoxycarbonylamino-C₁₋₆alkoxy, C₁₋₆alkoxycarbonylamino-C₁₋₆alkyl, C₁₋₆alkyl, (N-C₁₋₆alkyl)-C₁₋₆alkoxy-C₁₋₆alkylcarbamoyl, (N-C₁₋₆alkyl)-C₁₋₆alkoxy-C₁₋₆alkylcarbonylamino, (N-C₁₋₆alkyl)-C₁₋₆alkoxy-carbonylamino, (N-C₁₋₆alkyl)-C₀₋₆alkylcarbonylamino-C₁₋₆alkoxy, (N-C₁₋₆alkyl)-C₀₋₆alkylcarbonylamino-C₁₋₆alkyl, (N-C₁₋₆alkyl)-C₁₋₆alkylsulphonylamino-C₁₋₆alkoxy, (N-C₁₋₆alkyl)-C₁₋₆alkylsulphonylamino-C₁₋₆alkyl, C₁₋₆alkylamidinyl, C₁₋₆alkylaminocarbonyl-C₁₋₆alkoxy, di-C₁₋₆alkylaminocarbonyl-C₁₋₆alkoxy, C₁₋₆alkylaminocarbonyl-C₁₋₆alkoxy-C₁₋₆alkyl, C₁₋₆alkylaminocarbonyl-C₁₋₆alkyl, C₁₋₆alkylamino-carbonylamino-C₁₋₆alkoxy, C₁₋₆alkylaminocarbonylamino-C₁₋₆alkyl, di-C₁₋₆alkylaminocarbonyl-C₁₋₆alkyl, C₁₋₆alkylamino-C₂₋₆alkoxy, di-C₁₋₆alkylamino-C₂₋₆alkoxy, C₁₋₆alkylamino-C₁₋₆alkyl, di-C₁₋₆alkylamino-C₁₋₆alkyl, C₁₋₆alkylcarbamoyl, di-C₁₋₆alkylcarbamoyl, C₀₋₆alkylcarbonylamino-C₁₋₆alkoxy, C₀₋₆alkylcarbonylamino, C₀₋₆alkylcarbonylamino-C₁₋₆alkyl, C₁₋₆alkylcarbonyloxy-C₁₋₆alkoxy, C₁₋₆alkylcarbonyloxy-C₁₋₆alkyl, C₁₋₆alkylsulphonyl, C₁₋₆alkylsulphonyl-C₁₋₆alkoxy, C₁₋₆alkylsulphonyl-C₁₋₆alkyl, C₁₋₆alkylsulphonylamino-C₁₋₆alkoxy, C₁₋₆alkylsulphonylamino-C₁₋₆alkyl, carbamoyl, carbamoyl-C₁₋₆alkoxy, carbamoyl-C₁₋₆alkyl, carboxy-C₁₋₆alkoxy, carboxy-C₁₋₆alkoxy-C₁₋₆alkyl, carboxy-C₁₋₆alkyl, cyano, cyano-C₁₋₆alkoxy, cyano-C₁₋₆alkyl, C₃₋₆cycloalkylcarbonyl-amino-C₁₋₆alkoxy, C₃₋₆cycloalkylcarbonylamino-C₁₋₆alkyl, cyclopropyl-C₁₋₆alkyl, O,N-dimethylhydroxylamino-C₁₋₆alkyl, halogen, hydroxy-C₂₋₆alkoxy-C₁₋₆alkoxy, hydroxy-C₂₋₆alkoxy-C₁₋₆alkyl, hydroxy-C₁₋₆alkyl, (N-hydroxy)-C₁₋₆alkylaminocarbonyl-C₁₋₆alkoxy, (N-hydroxy)-C₁₋₆alkylaminocarbonyl-C₁₋₆alkyl, (N-hydroxy)aminocarbonyl-C₁₋₆alkoxy, (N-hydroxy)aminocarbonyl-C₁₋₆alkyl, 2-oxooxazolidinyl-C₁₋₆alkoxy, 2-oxooxazolidinyl-C₁₋₆alkyl, O-methyloximyl-C₁₋₆alkyl or trifluoromethyl; or
(E) R¹ is aryl which is substituted by 3-acetamidomethylpyrrolidinyl, 3-C₁₋₆alkoxy-C₁₋₆alkyl-pyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolylalkoxy, imidazolylalkyl, 2-methylimidazolylalkoxy, 2-methylimidazolylalkyl, 3-methyl-[1,2,4]-oxadiazol-5-ylalkoxy, 5-methyl-[1,2,4]-oxadiazol-3-ylalkoxy, 3-methyl-[1,2,4]-oxadiazol-5-ylalkyl, 5-methyl-[1,2,4]-oxadiazol-3-ylalkyl, 4-methylpiperazinyl, 5-methyltetrazol-1-ylalkoxy, 5-methyltetrazol-1-ylalkyl, morpholinyl, [1,2,4]-oxadiazol-5-ylalkoxy, [1,2,4]-oxadiazol-5-ylalkyl, oxazol-4-ylalkoxy, oxazol-4-ylalkyl, 2-oxo-[1,3]oxazinyl, 2-oxooxazolidinyl, 2-oxoimidazolidinyl, 2-oxopyrrolidinyl, 4-oxopiperidinyl, 2-oxopyrrolidinylalkoxy, 2-oxopyrrolidinylalkyl, 2-oxotetrahydropyrimidinyl, 4-oxothiomorpholinyl, piperazinyl, piperidinyl, pyrrolidinyl, pyrrolyl, [1,2,4]-triazol-1-ylalkoxy, [1,2,4]-triazol-4-ylalkoxy, [1,2,4]-triazol-1-ylalkyl, [1,2,4]-triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5-ylalkyl, thiazol-4-ylalkoxy, thiazol-4-ylalkyl, thiomorpholinyl; or
(F) R¹ is heterocyclyl optionally substituted as indicated under (D) or (E), in particular benzo[1,3]dioxolyl, benzofuranyl, benzooxazolyl, dihydrobenzofuranyl, 3,4-dihydro-2H-benzo[1,4]oxazinyl, dihydro-3H-benzo[1,4]oxazinyl, dihydro-2H-benzo[1,4]thiazinyl, 2,3-dihydroindolyl, dihydro-1 H-pyrido[2,3-b][1,4]oxazinyl, 1,1-dioxodihydro-2H-benzo[1,4]thiazinyl, indazolyl, indolyl, [1,5]naphthyridyl, oxazolyl, 2-oxoazepanyl, 3-oxo-4H-benzo[1,4]oxazinyl, 2-oxobenzooxazolyl, 3-oxo-4H-benzo[1,4]thiazinyl, 2-oxodihydrobenzo[e][1,4]diazepinyl, 2-oxodihydrobenzo-[d][1,3]oxazinyl, 2-oxodihydro-1H-quinazolinyl, 4-oxodihydroimidazolyl, 2-oxo-1,3-dihydroindolyl, 1-oxo-3H-isobenzofuranyl, 2-oxopiperidinyl, 2-oxo-1 H-pyrido[2,3-b][1,4]oxazinyl, 1-oxopyridyl, 2-oxotetrahydrobenzo[e][1,4]diazepinyl, 4-oxo-3H-thieno[2,3-d]pyrimidinyl, 5-oxo-4H-[1,2,4]triazinyl, phthalazinyl, pyrazolyl, 1H-pyrrolizinyl, 1 H-pyrrolo[2,3-b]pyridyl, pyrrolyl, tetrahydroquinoxalinyl, tetrahydropyranyl, triazinyl or 1,1,3-trioxodihydro-2H-1lambda*6*-benzo[1,4]thiazinyl;

R² is phenyl or a heterocyclyl which is linked via a C atom, each of which radicals may be substituted by 1-4 C₁₋₆alkanoyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylenedioxy, C₁₋₆alkylsulphanyl, C₁₋₆alkylsulphonyl, optionally N-mono- or N,N-di-C₁₋₆-alkylated amino, optionally N-mono- or N,N-di-C₁₋₆-alkylated carbamoyl, optionally esterified carboxy, cyano, cyano-C₁₋₆alkyl, C₃₋₈cycloalkoxy, C₃₋₈cycloalkyl, halogen, hydroxy-C₁₋₆alkyl, nitro, oxide, oxo, trifluoromethyl, trifluoromethoxy or optionally N-C₁₋₆-alkylated piperazinyl-C₁₋₆alkyl;
R³ is hydrogen, hydroxy, C₁₋₆alkoxy or C₂₋₆alkenyloxy;
R⁴ is C₁₋₆alkoxy, C₁₋₆alkoxy-C₁₋₆alkoxy, optionally N-mono- or N,N-di-C₁₋₆-alkylated amino-C₁₋₆alkoxy, optionally N-C₁₋₆-alkylated C₁₋₆alkoxycarbonylamino-C₁₋₆alkoxy, optionally N-C₁₋₆-alkylated C₁₋₆alkylcarbonylamino-C₁₋₆alkoxy, C₃₋₈cycloalkyloxy, C₃₋₈cycloalkyloxy-C₁₋₆alkoxy, hydroxy, hydroxy-C₂₋₆alkoxy, hydroxy-C₂₋₆alkoxy-C₁₋₆alkoxy, heterocyclyl-C₁₋₆alkoxy, heterocyclyloxy, heterocyclyloxy-C₁₋₆alkoxy or oxo;
R⁵ is acyl, C₂₋₈alkenyl, C₁₋₆alkyl, aryl-C₁₋₆alkyl or hydrogen;
R⁶ is acyl, C₁₋₆alkoxy-C₁₋₆alkyl, C₁₋₆alkyl or aryl-C₁₋₆alkyl or hydrogen;
R⁷ is C₁₋₆alkoxycarbonyl-C₁₋₆alkyl, C₁₋₆alkyl, carboxy-C₁₋₆alkyl or hydrogen;
W is oxygen, methylene or difluoromethylene;
X is a bond, oxygen or sulphur, where the bond originating from an oxygen or sulphur atom leads to a saturated C atom of group Z or to R¹, or a group >CH-R⁵, >CHOR⁶, -O-CO-, >CO, >C=NOR⁷, -O-CHR⁵- or -O-CHR⁵-CO-NR⁶-;
Z is C₁₋₆alkylene, C₂₋₈alkenylene, hydroxy-C₁₋₆alkylidene, -O-, -S-, -O-alk-, -S-alk-, -alk-O-, -alk-S- or-alk-NR⁶-, where alk is C₁₋₆alkylene; and where
   (a) if Z is -O-alk- or -S-alk-, then X is -CHR⁵-; and
   (b) if X is a bond, then Z is C₂₋₈alkenylene, -alk-O- or -alk-S-;
n is 1 or, if X is -O-CO- or -O-CHR⁵-CO-NR⁶-, 0 or 1;
and salts, especially pharmaceutically acceptable salts thereof.

Examples of C₁₋₆alkyl and alkoxy radicals are methyl, ethyl, n-propyl, isopropyl, n-butyl, isobutyl, sec-butyl, tert-butyl, pentyl, hexyl, and methoxy, ethoxy, propoxy, isopropoxy, butoxy, isobutoxy, sec-butoxy and tert-butoxy. C₁₋₆Alkylenedioxy radicals are preferably methylenedioxy, ethylenedioxy and propylenedioxy. Examples of C₁₋₆alkanoyl radicals are acetyl, propionyl and butyryl. Cycloalkyl is a saturated, cyclic hydrocarbon radical having 3 to 12 carbon atoms, for example cyclopropyl, cyclobutyl, cyclopentyl, cyclohexyl, cycloheptyl, bicyclo[2.2.1]heptyl, cyclooctyl, bicyclo[2.2.2]octyl and adamantyl. C₁₋₈Alkylene radicals are, for example, methylene, ethylene, propylene, 2-methylpropylene, tetra-, penta- and hexamethylene; C₂₋₈alkenylene radicals are, for example, vinylene and propenylene; C₂₋₈alkynylene radical is, for example, ethynylene; acyl radicals are alkanoyl radicals, preferably C₁₋₆alkanoyl radicals, or aroyl radicals such as benzoyl. Aryl refers to mono- or polynuclear aromatic radicals which may be substituted one or more times, such as, for example, phenyl, substituted phenyl, naphthyl, substituted naphthyl, tetrahydronaphthyl or substituted tetrahydronaphthyl. Examples of substituents on such aryl radicals are C₁₋₆alkyl, trifluoromethyl, nitro, amino, C₂₋₈alkenyl, C₁₋₆alkoxy, C₁₋₆alkylcarbonyloxy, hydroxy, halogen, cyano, carbamoyl, carboxy and C₁₋₆alkylenedioxy, and optionally halogen-, C₁₋₆alkyl-, C₁₋₆alkoxy- or dihydroxy-C₁₋₆alkylaminocarbonyl-substituted phenyl, phenoxy, phenylthio, phenyl-C₁₋₆alkyl or phenyl-C₁₋₆alkoxy. Further examples of substituents on aryl or heterocyclyl radicals are C₁₋₆alkoxycarbonylphenyl, hydroxy-C₁₋₆alkylphenyl, benzyloxy, pyridylcarbonyl-amino-C₁₋₆alkyl, C₂₋₆alkenyloxy, C₁₋₆alkoxy-C₁₋₆alkoxy, C₁₋₆alkoxy-C₁₋₆alkoxy-C₁₋₆alkyl, methoxybenzyloxy, hydroxybenzyloxy, phenethyloxy, methylenedioxybenzyloxy, dioxolanyl-C₁₋₆alkoxy, cyclopropyl-C₁₋₆alkyl, cyclopropyl-C₁₋₆alkoxy, hydroxy-C₂₋₆alkoxy, carbamoyloxy-C₁₋₆alkoxy, pyridylcarbamoyloxy-C₁₋₆alkoxy, benzoyloxy-C₁₋₆alkoxy, C₁₋₆alkoxycarbonyl, C₀₋₆alkylcarbonylamino, C₀₋₆alkylcarbonylamino-C₁₋₆alkyl, C₀₋₆alkylcarbonylamino-C₁₋₆alkoxy, (N-C₁₋₆alkyl)-C₀₋₆alkylcarbonylamino-C₁₋₆alkyl, (N-C₁₋₆alkyl)-C₀₋₆alkylcarbonylamino-C₁₋₆alkoxy, C₃₋₆cycloalkylcarbonylamino-C₁₋₆alkyl, C₃₋₆cycloalkylcarbonylamino-C₁₋₆alkoxy, C₁₋₆alkoxy-C₁₋₆alkyl, hydroxy-C₁₋₆alkyl, hydroxy-C₂₋₆alkoxy-C₁₋₆alkyl, hydroxy-C₂₋₆alkoxy-C₁₋₆alkoxy, C₁₋₆alkoxycarbonylamino-C₁₋₆alkyl, C₁₋₆alkoxycarbonylamino-C₁₋₆alkoxy, C₁₋₆alkyl-aminocarbonylamino-C₁₋₆alkyl, C₁₋₆alkylaminocarbonylamino-C₁₋₆alkoxy, C₁₋₆alkylaminocarbonyl-C₁₋₆alkyl, C₁₋₆alkylaminocarbonyl-C₁₋₆alkoxy, C₁₋₆alkylaminocarbonyl-C₁₋₆alkoxy-C₁₋₆alkyl, di-C₁₋₆alkylaminocarbonyl-C₁₋₆alkyl, di-C₁₋₆alkylaminocarbonyl-C₁₋₆alkoxy, C₁₋₆alkylcarbonyloxy-C₁₋₆alkyl, C₁₋₆alkylcarbonyloxy-C₁₋₆alkoxy, cyano-C₁₋₆alkyl, cyano-C₁₋₆alkoxy, 2-oxooxazolidinyl-C₁₋₆alkyl, 2-oxooxazolidinyl-C₁₋₆alkoxy, C₁₋₆alkoxycarbonyl-C₁₋₆alkyl, C₁₋₆alkoxycarbonyl-C₁₋₆alkoxy, C₁₋₆alkylsulphonylamino-C₁₋₆alkyl, C₁₋₆alkylsulphonylamino-C₁₋₆alkoxy, (N-C₁₋₆alkyl)-C₁₋₆alkylsulphonylamino-C₁₋₆alkyl, (N-C₁₋₆alkyl)-C₁₋₆alkylsulphonylamino-C₁₋₆alkoxy, C₁₋₆alkylamino-C₁₋₆alkyl, C₁₋₆alkylamino-C₂₋₆alkoxy, di-C₁₋₆alkylamino-C₁₋₆alkyl, di-C₁₋₆alkylamino-C₂₋₆alkoxy, C₁₋₆alkylsulphonyl-C₁₋₆alkyl, C₁₋₆alkylsulphonyl-C₁₋₆alkoxy, carboxy-C₁₋₆alkyl, carboxy-C₁₋₆alkoxy, carboxy-C₁₋₆alkoxy-C₁₋₆alkyl, C₁₋₆alkoxy-C₁₋₆alkylcarbonyl, acyl-C₁₋₆alkoxy-C₁₋₆alkyl, (N-C₁₋₆alkyl)-C₁₋₆alkoxycarbonylamino, (N-hydroxy)-C₁₋₆alkylaminocarbonyl-C₁₋₆alkyl, (N-hydroxy)-C₁₋₆alkylaminocarbonyl-C₁₋₆alkoxy, (N-hydroxy)aminocarbonyl-C₁₋₆alkyl, (N-hydroxy)aminocarbonyl-C₁₋₆alkoxy, C₁₋₆alkoxyamino-carbonyl-C₁₋₆alkyl, 6-alkoxyaminocarbonyl-C₁₋₆alkoxy, (N-C₁₋₆alkoxy)-C₁₋₆alkylaminocarbonyl-C₁₋₆alkyl, (N-C₁₋₆alkoxy)-C₁₋₆alkylaminocarbonyl-C₁₋₆alkoxy, (N-acyl)-C₁₋₆alkoxy-C₁₋₆alkylamino, C₁₋₆alkoxy-C₁₋₆alkylcarbamoyl, (N-C₁₋₆alkyl)-C₁₋₆alkoxy-C₁₋₆alkylcarbamoyl, C₁₋₆alkoxy-C₁₋₆alkylcarbonyl, C₁₋₆alkoxy-C₁₋₆alkylcarbonylamino, (N-C₁₋₆alkyl)-C₁₋₆alkoxy-C₁₋₆alkylcarbonylamino, 1 -C₁₋₆alkoxy-C₁₋₆alkylimidazol-2-yl, 1-C₁₋₆alkoxy-C₁₋₆alkyltetrazol-5-yl, 5-C₁₋₆alkoxy-C₁₋₆alkyltetrazol-1-yl, 2-C₁₋₆alkoxy-C₁₋₆alkyl-4-oxoimidazol-1-yl, carbamoyl-C₁₋₆alkyl, carbamoyl-C₁₋₆alkoxy, C₁₋₆alkylcarbamoyl, di-C₁₋₆alkylcarbamoyl, C₁₋₆alkylsulphonyl, C₁₋₆alkylamidinyl, acetamidinyl-C₁₋₆alkyl, O-methyloximyl-C₁₋₆alkyl, O,N-dimethylhydroxyl-amino-C₁₋₆alkyl, C₃₋₆cycloalkyl-C₁₋₆alkanoyl, aryl-C₁₋₆alkanoyl, heterocyclyl-C₁₋₆alkanoyl; and optionally halogen-, C₁₋₆alkyl-, C₁₋₆alkoxy- or dihydroxy-C₁₋₆alkylaminocarbonyl-substituted pyridyl, pyridyloxy, pyridylthio, pyridylamino, pyridyl-C₁₋₆alkyl, pyridyl-C₁₋₆alkoxy, pyrimidinyl, pyrimidinyloxy, pyrimidinylthio, pyrimidinylamino, pyrimidinyl-C₁₋₆alkyl, pyrimidinyl-C₁₋₆alkoxy, thienyl, thienyl-C₁₋₆alkyl, thienyl-C₁₋₆alkoxy, furyl, furyl-C₁₋₆alkyl , furyl-C₁₋₆alkoxy.

The term heterocyclyl refers to mono-, bi- or tricyclic, saturated and unsaturated heterocyclic radicals having 1 to 4 nitrogen and/or 1 or 2 sulphur or oxygen atoms, which may be substituted one or more times, in particular by (in the case of unsaturated heterocyclyl radicals) alkyl, hydroxy, alkoxy, nitro or halogen or by substituents as defined above for aryl radicals, or (in the case of saturated heterocyclyl radicals) may be substituted by alkyl or alkoxy.

Examples of heterocyclyl radicals are pyridyl, thienyl, pyrazinyl, triazolyl, imidazolyl, benzothiazolyl, furyl, pyranyl, tetrahydropyranyl, azetidinyl, pyrimidinyl, morpholinyl, quinazolinyl, quinolyl, quinoxalinyl, isoquinolyl, benzo[b]thienyl, isobenzofuranyl, benzoimidazolyl, 2-oxobenzimidazolyl, oxazolyl, thiazolyl, indolyl, pyrrolyl, 2-oxodihydro-benzo-[d][1,3]oxazinyl, 4-oxodihydroimidazolyl, 5-oxo-4H-[1,2,4]triazinyl, 3-oxo-4H-benzo[1,4]thiazinyl, tetrahydroquinoxalinyl, 2-oxodihydro-1 H-quinazolinyl, 1,1,3-trioxo-dihydro-2H-1lambda*6*-benzo[1,4]thiazinyl, 1-oxopyridyl, 3,4-dihydro-2H-benzo[1,4]oxazinyl, dihydro-3H-benzo[1,4]oxazinyl, 2-oxotetrahydrobenzo[e][1,4]diazepinyl, 2-oxodihydro-benzo[e][1,4]diazepinyl, 1 H-pyrrolizinyl, phthalazinyl, 1-oxo-3H-isobenzofuranyl, 4-oxo-3H-thieno[2,3-d]pyrimidinyl, 3-oxo-4H-benzo[1,4]oxazinyl, [1,5]naphthyridyl, [1,7]naphthyridyl, [1,8]naphthyridyl, dihydro-2H-benzo[1,4]thiazinyl, 1,1-dioxodihydro-2H-benzo[1,4]thiazinyl, 2-oxo-1 H-pyrido[2,3-b][1,4]oxazinyl, dihydro-1 H-pyrido[2,3-b][1,4]oxazinyl, 1 H-pyrrolo[2,3-b]pyridyl, 3H-imidazo[4,5-c]pyridyl, 1 H-pyrrolo[3,2-c]pyridyl, benzo[1,3]dioxolyl, benzooxazolyl, 2-oxobenzooxazolyl, 2-oxo-1,3-dihydroindolyl, 2,3-dihydroindolyl, spiro[cyclopropyl-1,3'-(2-oxo-1,3-dihydroindolyl), indazolyl or benzofuranyl. Examples of substituted heterocyclyl radicals are nitrobenzothiazolyl, phenyltetrazolyl, phenyloxadiazolyl, phenylpiperidinyl, phenylpiperazinyl, phenylpyrrolidinyl, thienyloxadiazolyl, furanyloxadiazolyl, benzyloxadiazolyl or phenyloxazolyl. Examples of saturated heterocyclyl radicals are dioxolanyl, dioxanyl, dithiolanyl, dithianyl, pyrrolidinyl, piperidinyl, piperazinyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, tetrahydropyranyl, 2-oxoimidazolidinyl, 2-oxooxazolidinyl, 2-oxopiperidinyl, 2-oxopyrrolidinyl, 2-oxo-[1,3]oxazinyl, 2-oxoazepanyl, 2-oxotetrahydropyrimidinyl and the like.

In the case of R¹, the aryl, aroyl and heterocyclyl radicals may additionally be substituted by heterocyclylalkyl, heterocyclylalkoxy, heterocyclylalkoxyalkyl or heterocyclyl such as, for example, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]-triazol-1-ylalkyl, [1,2,4]-triazol-1-ylalkoxy, [1,2,4]-triazol-4-ylalkyl, [1,2,4]-triazol-4-ylalkoxy, [1,2,4]-oxadiazol-5-ylalkyl, [1,2,4]-oxadiazol-5-ylalkoxy, 3-methyl-[1,2,4]-oxadiazol-5-ylalkyl, 3-methyl-[1,2,4]-oxadiazol-5-ylalkoxy, 5-methyl-[1,2,4]-oxadiazol-3-ylalkyl, 5-methyl-[1,2,4]-oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyltetrazol-1-ylalkyl, 5-methyltetrazol-1-ylalkoxy, thiazol-4-ylalkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy or N-methylpiperazino-alkyl, N-methylpiperazinoalkoxy, N-methylpiperazinoalkoxyalkyl, and alkylaminoalkyl, alkylaminoalkoxy, alkylaminoalkoxyalkyl, mono- and polyhydroxyalkyl, -alkoxy, -alkoxyalkyl and -alkoxyalkoxy, carbamoylalkyloxy, C₁₋₆alkoxy, amino-C₁₋₆alkoxy, hydroxy-C₂₋₆alkoxy, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₆alkoxy-C₁₋₆alkyl-pyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo-[1,3]oxazinyl, 2-oxotetrahydropyrimidinyl and the like or by the radical-O-CH₂CH(OH)CH₂NRx , where NRx is a mono- or di-C₁₋₆alkylamino, piperidino, morpholino, piperazino or N-methylpiperazino radical.

The term polyhydroxyalkyl refers to C₁₋₇alkyl radicals which may be substituted by 2-6 hydroxy groups, such as, for example, glyceryl, arabityl, sorbityl etc.

The compounds of the formula (I) have at least two asymmetric carbon atoms and can therefore exist in the form of optically pure diastereomers, mixtures of diastereomers, diastereomeric racemates, mixtures of diastereomeric racemates or as meso compounds. The invention includes all these forms.

Mixtures of diastereomers, diastereomeric racemates or mixtures of diastereomeric racemates can be fractionated by conventional methods, e.g. by column chromatography, thin-layer chromatography, HPLC and the like.

Salts of compounds with salt-forming groups are in particular acid addition salts, salts with bases or, if a plurality of salt-forming groups is present, optionally also mixed salts or inner salts.

Salts are primarily the pharmaceutically acceptable or non-toxic salts of compounds of the formula (I).

Such salts are formed for example by compounds of the formula (I) having an acidic group, e.g. a carboxy or sulpho group, and are for example their salts with suitable bases, such as non-toxic metal salts derived from metals of group la, Ib, IIa and IIb of the Periodic Table of the Elements, e.g. alkali metal, in particular lithium, sodium or potassium, salts, alkaline earth metal salts, for example magnesium or calcium salts, furthermore zinc salts or ammonium salts, also salts formed with organic amines such as optionally hydroxy-substituted mono-, di- or trialkylamines, especially mono-, di- or tri-lower-alkylamines, or with quaternary ammonium bases, e.g. methyl-, ethyl-, diethyl- or triethylamine, mono-, bis- or tris(2-hydroxy-lower-alkyl)amines such as ethanol-, diethanol- or triethanolamine, tris(hydroxymethyl)methylamine or 2-hydroxy-tertiary-butylamine, N,N-di-lower-alkyl-N-(hydroxy-lower-alkyl)amine, such as N,N-dimethyl-N-(2-hydroxyethyl)amine, or N-methyl-D-glucamine, or quaternary ammonium hydroxides such as tetrabutylammonium hydroxide. The compounds of the formula I having a basic group, e.g. an amino group, can form acid addition salts, e.g. with suitable inorganic acids, e.g. hydrohalic acid such as hydrochloric acid, hydrobromic acid, sulphuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulphonic or phosphonic acids or N-substituted sulphamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, furthermore amino acids such as, for example, the α-amino acids mentioned hereinabove, and methanesulphonic acid, ethanesulphonic acid, 2-hydroxyethanesulphonic acid, ethane-1,2-disulphonic acid, benzenesulphonic acid, 4-toluenesulphonic acid, naphthalene-2-sulphonic acid, 2- or 3-phosphoglycerate, glucose 6-phosphate, N-cyclohexylsulphamic acid (to form cyclamates) or with other acidic organic compounds such as ascorbic acid. Compounds of the formula (I) having acidic and basic groups may also form inner salts.

Pharmaceutically unsuitable salts may also be used for isolation and purification.

Prodrug derivatives of the compounds described herein are derivatives thereof which on *in vivo* use liberate the original compound by a chemical or physiological process. A prodrug may for example be converted into the original compound when a physiological pH is reached or by enzymatic conversion. Possible examples of prodrug derivatives are esters of freely available carboxylic acids, S- and O-acyl derivatives of thiols, alcohols or phenols, the acyl group being defined as above. Preferred derivatives are pharmaceutically acceptable ester derivatives which are converted by solvolysis in physiological medium into the original carboxylic acid, such as, for example, lower alkyl esters, cycloalkyl esters, lower alkenyl esters, benzyl esters, mono- or disubstituted lower alkyl esters such as lower ω-(amino, mono- or dialkylamino, carboxy, lower alkoxycarbonyl) - alkyl esters or such as lower α-(alkanoyloxy, alkoxycarbonyl or dialkylaminocarbonyl) - alkyl esters; conventionally, pivaloyloxymethyl esters and similar esters are used as such.

Because of the close relationship between a free compound, a prodrug derivative and a salt compound, a particular compound in this invention also includes its prodrug derivative and salt form, where this is possible and appropriate.

The compounds of the formula (I) also include those compounds in which one or more atoms are replaced by their stable, non-radioactive isotopes; for example, a hydrogen atom by deuterium.

Preferred compounds of the invention are those of the general formula (IA) 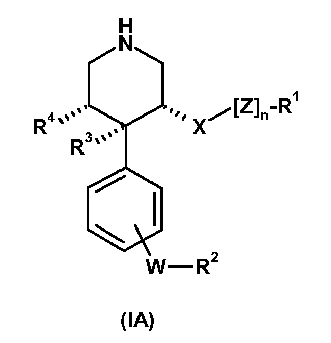 in which R¹, R², R³, R⁴, W, X, Z and n have the meaning indicated above for the compounds of the formula (I).

A further preferred group of compounds of the formula (I), or particularly preferably of the formula (IA), are compounds in which
R¹ is aryl under the conditions indicated for (A), (B) or (C), or heterocyclyl, substituted as indicated under (D) or (E), where heterocyclyl is particularly preferably selected from benzo[1,3]dioxolyl, benzofuranyl, benzooxazolyl, dihydrobenzofuranyl, 3,4-dihydro-2H-benzo[1,4]oxazinyl, dihydro-3H-benzo[1,4]oxazinyl, dihydro-2H-benzo[1,4]thiazinyl, 2,3-dihydroindolyl, dihydro-1H-pyrido[2,3-b][1,4]oxazinyl, 1,1-dioxodihydro-2H-benzo[1,4]thiazinyl, indazolyl, indolyl, [1,5]naphthyridyl, oxazolyl, 2-oxoazepanyl, 3-oxo-4H-benzo[1,4]oxazinyl, 2-oxobenzooxazolyl, 3-oxo-4H-benzo[1,4]thiazinyl, 2-oxodihydro-benzo[e][1,4]diazepinyl, 2-oxodihydrobenzo-[d][1,3]oxazinyl, 2-oxodihydro-1 H-quinazolinyl, 4-oxodihydroimidazolyl, 2-oxo-1,3-dihydroindolyl, 1-oxo-3H-isobenzofuranyl, 2-oxopiperidinyl 2-oxo-1 H-pyrido[2,3-b][1,4]oxazinyl, 1-oxopyridyl, 2-oxotetrahydrobenzo[e][1,4]diazepinyl, 4-oxo-3H-thieno[2,3-d]pyrimidinyl, 5-oxo-4H-[1,2,4]triazinyl, phthalazinyl, pyrazolyl, 1H-pyrrolizinyl, 1 H-pyrrolo[2,3-b]pyridyl, pyrrolyl, tetrahydroquinoxalinyl, tetrahydropyranyl, triazinyl and 1,1,3-trioxodihydro-2H-1lambda*6*-benzo[1,4]thiazinyl.

A further preferred group of compounds of the formula (I), or particularly preferably of the formula (IA), are compounds in which
R¹ has the meaning as indicated for (A), (B), (C), (D), (E) or (F), particularly preferably as indicated for (B), (D), (E) or (F);
R² is phenyl or a heterocyclyl linked via a C atom, each of which radicals may be substituted by 1-4 C₁₋₆alkanoyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylenedioxy, C₁₋₆alkylsulphanyl, C₁₋₆alkylsulphonyl, optionally N-mono- or N,N-di-C₁₋₆-alkylated amino, optionally N-mono- or N,N-di-C₁₋₆-alkylated carbamoyl, optionally esterified carboxy, cyano, cyano-C₁₋₆alkyl, C₃₋₈cycloalkoxy, C₃₋₈cycloalkyl, halogen, hydroxy-C₁₋₆alkyl, nitro, oxide, oxo, trifluoromethyl, trifluoromethoxy or optionally N-C₁₋₆-alkylated piperazinyl-C₁₋₆alkyl;
R³ is hydrogen, hydroxy, C₁₋₆alkoxy or C₂₋₆alkenyloxy;
R⁴ is C₁₋₆alkoxy, C₁₋₆alkoxy-C₁₋₆alkoxy, optionally N-mono- or N,N-di-C₁₋₆-alkylated amino-C₁₋₆alkoxy, optionally N-C₁₋₆-alkylated C₁₋₆alkoxycarbonylamino-C₁₋₆alkoxy, optionally N-C₁₋₆-alkylated C₁₋₆alkylcarbonylamino-C₁₋₆alkoxy, C₃₋₈cycloalkyloxy, C₃₋₈cycloalkyloxy-C₁₋₆alkoxy, hydroxy, hydroxy-C₂₋₆alkoxy, hydroxy-C₂₋₆alkoxy-C₁₋₆alkoxy, heterocyclyl-C₁₋₆alkoxy, heterocyclyloxy, heterocyclyloxy-C₁₋₆alkoxy or oxo;
R⁵ is acyl, C₂₋₈alkenyl, C₁₋₆alkyl, aryl-C₁₋₆alkyl or hydrogen;
R⁶ is acyl, C₁₋₆alkoxy-C₁₋₆alkyl, C₁₋₆alkyl or aryl-C₁₋₆alkyl or hydrogen;
R⁷ is C₁₋₆alkoxycarbonyl-C₁₋₆alkyl, C₁₋₆alkyl, carboxy-C₁₋₆alkyl or hydrogen;
X is a bond, oxygen or sulphur, where the bond originating from an oxygen or sulphur atom leads to a saturated C atom of the group Z or to R¹, or a group >CH-R⁵, >CHOR⁶, -O-CO-, >CO, >C=NOR⁷, -O-CHR⁵- or-O-CHR⁵-CO-NR⁶-;
Z is C₁₋₆alkylene, C₂₋₈alkenylene, hydroxy-C₁₋₆alkylidene, -O-, -S-, -O-alk-, -S-alk-, -alk-O-, -alk-S-or-alk-NR⁶-, where alk is C₁₋₆alkylene, and where
   (a) if Z is -O-alk- or -S-alk-, then X is -CHR⁵-, and
   (b) if X is a bond, then Z is C₂₋₈alkenylene, -alk-O- or-alk-S-;
n is 1 is or, if X is -O-CO- or -O-CHR⁵-CO-NR⁶-, 0 or 1;
and pharmaceutically acceptable salts thereof.

Further preferred compounds of the formulae (I) and (IA) are those in which X is preferably oxygen, sulphur, -O-CHR⁵-, -O-CHR⁵-CO-NR⁶- or -CO-; Z is preferably methylene or-alk-O-.

Further preferred compounds of the formulae (I) and (IA) are those in which W is oxygen.

Further preferred compounds of the formulae (I) and (IA) are those in which R³ is hydrogen.

Further preferred compounds of the formulae (I) and (IA) are those in which R⁴ is optionally N-mono- or N,N-di-C₁₋₆-alkylated amino-C₁₋₆alkoxy or hydroxyl.

A group of preferred radicals R¹ includes the abovementioned substituted phenyl and naphthyl radicals, and tetrahydronaphthyl and methyl-substituted tetrahydronaphthyl.

Likewise preferred radicals R¹ are pyridyl, benzoimidazolyl, pyrimidinyl, 2- and 5-benzo[b]thienyl, 6- and 7-quinolyl, 6- and 7-isoquinolyl, 6- and 7-tetrahydroquinolyl, 6- and 7-tetrahydroisoquinolyl, 6-quinoxalinyl, 6- and 7-quinazolinyl, indolyl, 3,4-dihydro-2H-benzo[1,4]oxazinyl, dihydro-3H-benzo[1,4]oxazinyl, 3-oxo-4H-benzo[1,4]oxazinyl, benzooxazolyl, 2,3-dihydroindolyl, indazolyl or benzofuranyl, and halogen-, oxide-, oxo-, C₁₋₆alkoxy-, C₁₋₆alkoxy-C₁₋₆alkoxy-, C₁₋₆alkoxy-C₁₋₆alkyl-, C₁₋₆alkoxycarbonylamino-C₁₋₆alkoxy-, C₁₋₆alkoxycarbonylamino-C₁₋₆alkyl-, C₁₋₆alkyl-, C₀₋₆alkylcarbonylamino-C₁₋₆alkoxy-, C₀₋₆alkylcarbonylamino-C₁₋₆alkyl-, C₁₋₆alkoxy-C₁₋₆alkoxy-C₁₋₆alkyl-, cyano-C₁₋₆alkoxy-, cyano-C₁₋₆alkyl- or trifluoromethyl-substituted 6- and 7-quinolyl, 6- and 7-isoquinolyl, 6- and 7-tetrahydroquinolyl, 6- and 7-tetrahydroisoquinolyl, 6-quinoxalinyl, 6- and 7-quinazolinyl, indolyl, 3,4-dihydro-2H-benzo[1,4]oxazinyl, dihydro-3H-benzo[1,4]oxazinyl, 3-oxo-4H-benzo[1,4]oxazinyl, benzooxazolyl, 2,3-dihydroindolyl, indazolyl or benzofuranyl.

R¹ is very particularly preferably substituted 3,4-dihydro-2H-benzo[1,4]oxazinyl.

Preferred radicals R² in the meaning of a heterocyclyl linked via a C atom are pyridyl, pyrimidinyl, pyrazinyl, benzo[b]thienyl, quinolyl, isoquinolyl, quinoxalinyl, indolyl, 3,4-dihydro-2H-benzo[1,4]oxazinyl, 2,3-dihydrobenzofuranyl or benzofuranyl.

Preferred radicals R² are phenyl or a heterocyclyl linked via a C atom, which radicals may be substituted by 1-4 C₁₋₆alkanoyl, C₁₋₆alkoxy, C₁₋₆alkoxy-C₁₋₆alkoxy, C₁₋₆alkoxy-C₁₋₆alkyl, C₁₋₆alkyl, C₀₋₆alkylcarbonylamino, C₁₋₆alkylenedioxy, C₁₋₆alkylsulphanyl, C₁₋₆alkylsulphonyl, optionally N-mono- or N,N-di-C₁₋₆-alkylated amino, optionally N-mono- or N,N-di-C₁₋₆-alkylated carbamoyl, cyano, halogen, hydroxy-C₁₋₆alkyl, oxide or trifluoromethyl.

The compounds of the formula (I) can be prepared in a manner analogous to preparation processes disclosed in the literature. Similar preparation processes are described for example in WO 97/09311. Details of the specific preparation variants can be found in the examples.

The compounds of the formula (I) can also be prepared in optically pure form. Separation into antipodes can take place by methods known per se, either preferably at an early stage in the synthesis by salt formation with an optically active acid such as, for example, (+)- or (-)-mandelic acid and separation of the diastereomeric salts by fractional crystallization or preferably at a rather late stage by derivatizing with a chiral auxiliary component such as, for example, (+)- or (-)-camphanoyl chloride, and separation of the diastereomeric products by chromatography and/or crystallization and subsequent cleavage of the linkage to the chiral auxiliary. The pure diastereomeric salts and derivatives can be analysed to determine the absolute configuration of the contained piperidine by conventional spectroscopic methods, with X-ray spectroscopy on single crystals representing a particularly suitable method.

The above-specified compound groups are not to be regarded as closed, but rather it is possible in a sensible manner, for example in order to replace general by more specific definitions, to exchange parts of these compound groups with one another or for the definitions given or to omit them.

The compounds of formula (I) and (IA), respectively, and their pharmaceutically useful salts reveal inhibitory activities on the enzymes beta-secretase, cathepsin D, plasmepsin II and/or HIV-protease.

The activitiy of inhibitors of beta-secretase, cathepsin D, plasmepsin II and/or HIV protease can be assessed experimentally with following *in vitro* assays.

The protease inhibitory activity of compounds can be tested with an assay kit using the fluorescence resonance energy transfer (FRET) technology and a recombinant i.e. baculovirus expressed enzyme preparation. The FRET is used to monitor the cleavage of the peptide substrate. The principle of the assay is as follows relies on a measurable energy difference, quantitatively depending on the presence of a peptide sequence. The peptide substrate is synthesized with two terminal fluorophores, a fluorescent donor and quenching acceptor. The distance between these two groups is selected so that upon light excitation, the donor fluorescence energy is significantly quenched by the acceptor through resonance energy transfer. Upon cleavage by the protease, the fluorophore is separated from the quenching group, restoring the fluorescence yield of the donor. Thus a weakly fluorescent peptide substrate becomes highly fluorescent upon enzymatic cleavage; the increase in fluorescence is linearly related to the rate of proteolysis.

The FRET assay was performed in white polysorp plates. The assay buffer consisted of 50 mM sodium acetate pH 5, 392 mM sodium chloride, 12.5% glycerol and 0.1% BSA. The incubates per well were composed of 160 µl buffer, 10 µl inhibitor in DMSO, 10 µl peptide substrate in DMSO and 20 µl enzyme-solution. The inhibitors are tested in a concentration range of 1 pM to 1 mM. The fluorescently marked donor and acceptor peptide substrates are generated by solid phase peptide synthesis (Applied Biosystems). The beta-secretase peptide substrate Rh-Glu-Val-Asn-Leu-Asp-Ala-Glu-Phe-Lys-Quencher is obtained from Invitrogen, Carlsbad, CA, USA. The cathepsin D peptide substrate of the sequence DABCYL-Pro-Thr-Glu-Phe-Phe-Arg-Leu-OXL, the plasmepsin peptide substrate of the sequence DABCYL-Glu-Arg-Nle-Phe-Leu-Ser-Phe-Pro-OXL and the HIV protease peptide substrate of the sequence DABCYL-His-Lys-Ala-Arg-Val-Leu-Tyr-Glu-Ala-Nle-Ser-EDANS are all obtained from AnaSpec Inc, San Jose, CA, USA. The recombinantly expressed enzyme preparations are added in various amounts to the assay systems eg the beta-sectrase concentration is 1 unit/ml incubation volume, the cathepsin D concentration is 100 ng/ml, the HIV protease concentration is 500 ng/ml and the plasmepsin II concentration is 50 ng/ml. The reaction is started upon addition of the enzyme solution. The incubation occurs at 37°C over 30-120 min ie specifically the beta-secretase incubation lasts 60 min, the cathepsin D incubation 120 min, the plasmepsin II incubation 40 min and the HIV protease incubation 40 min. The reactions are stopped by the addition of 20 µl of a 1.0 M Tris Base solution. The enzymatic substrate to product conversion is assessed by fluorescence measurements at 460 nm wave length.

### In vitro enzyme inhibitory activities

The compounds of the present invention revealed structure-dependent and enzyme-specific inhibitory activities. The inhibitory activities were measured as IC50 values. Thus the beta-secretase inhibitory activity ranged between 1 pM and 1 mM; the values for cathepsin D ranged between 1 pM and 1 mM, for plasmepsin II between 1 pM and 1 mM and for HIV-protease between 1 pM and 1 mM.

The compounds of the formula (I) or preferred formula (IA) and the pharmaceutically usable salts thereof may find use as medicines, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of the formula (I) and pharmaceutically usable salts thereof may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.

Suitable excipients for soft gelatine capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

Subject of the present invention is also the use of the compounds of formula (I) and (IA), respectively, and their pharmaceutically useful salts for the prevention, delay of progression or the treatment of Alzheimer Disease, malaria or HIV infection.
Subject of the present invention is also the use of the compounds of formula (I) and (IA), respectively, and their pharmaceutically useful salts for the manufacture of a medication for the prevention, delay of progression or the treatment of Alzheimer Disease, malaria or HIV infection.

Subject of the present invention is also the method for the prevention, delay of progression or the treatment of Alzheimer Disease, malaria or HIV infection, whereby a therapeutically effective dose of a compound of the general formula (I) or preferred formula (IA) or a pharmaceutically acceptable salt thereof is applied.
Subject of the present invention is also a pharmaceutical preparation that contains for the inhibition of beta-secretase, cathepsin D, plasmepsin and/or HIV-protease a compound of the general formula (I), or preferred of formula (IA) or a pharmaceutically acceptable salt thereof as well as commonly used ingredients.
Subject of the present invention is also a pharmaceutical preparation for the prevention, delay of progression or treatment of Alzheimer Disease, malaria and HIV infection that contains a compound of the general formula (I), or preferred of formula (IA) or a pharmaceutically acceptable salt thereof as well as commonly used ingredients.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

### Examples

The following examples illustrate the present invention. All temperatures are stated in degrees Celsius and pressures in mbar. Unless mentioned otherwise, the reactions take place at room temperature. The abbreviation "Rf = xx (A)" means for example that the Rf is found in solvent system A to be xx. The ratio of amounts of solvents to one another is always stated in parts by volume. Chemical names for final products and intermediates have been generated on the basis of the chemical structural formulae with the aid of the AutoNom 2000 (Automatic Nomenclature) programme. Unless mentioned otherwise, the absolute stereochemistry of the 3,4,5-trisubstituted piperidine unit is (3S,4S,5R), with the absolute stereochemistry of the 4-substituents being dependent on the relative priorities of the 3- and 5-substituents.

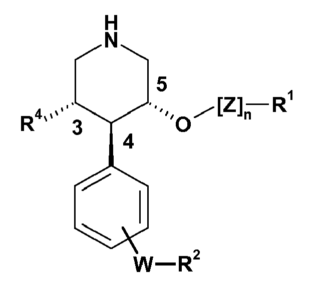

| **No.** | **Structure** | **Appearance** | **R_{f}(system)** | **Rt (method)** |
|---|---|---|---|---|
| 1 | 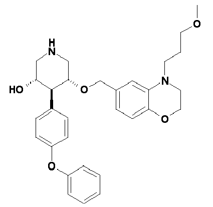 | colourless oil | 0.07 (A) | 4.04 (I) |
| 2 | 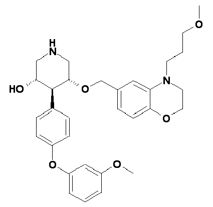 | beige resin | 0.32 (B) | 3.92 (I) |
| 3 | 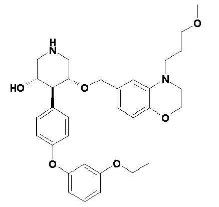 | yellow resin | 0.39 (B) | 4.28 (I) |
| 4 | 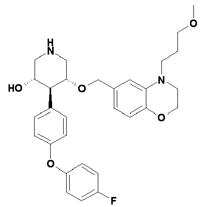 | brown oil | 0.37 (B) | 4.09 (I) |
| 5 | 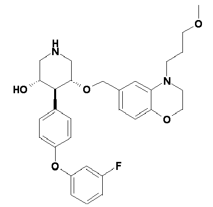 | yellow resin | 0.36 (B) | 4.11 (I) |
| 6 | 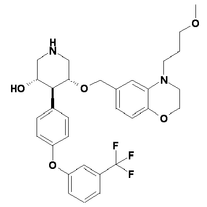 | colourless resin | 0.35 (B) | 4.44 (I) |
| 7 | 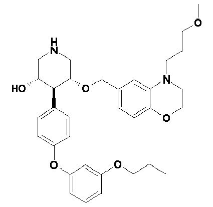 | colourless resin | 0.34 (B) | 4.58 (I) |
| 8 | 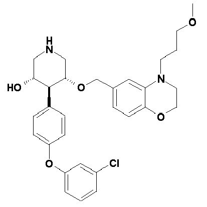 | colourless film | 0.19 (B) | 4.34 (I) |
| 9 | 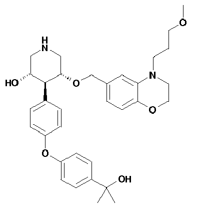 | colourless film | 0.15 (B) | 3.58 (I) |
| 10 | 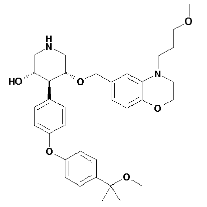 | colourless film | 0.36 (B) | 3.68 (I) |
| 11 | 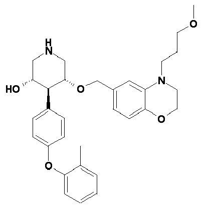 | beige resin | 0.30 (B) | 4.26 (I) |
| 12 | 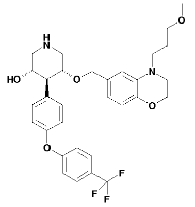 | beige oil | 0.24 (B) | 4.37 (I) |
| 13 | 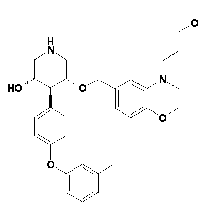 | beige oil | 0.28 (B) | 4.23 (I) |
| 14 | 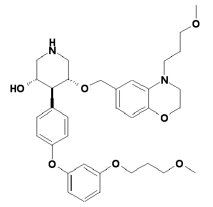 | colourless oil | 0.24 (B) | 4.13 (I) |
| 15 | 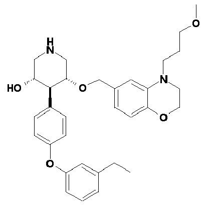 | colourless oil | 0.32 (B) | 4.46 (I) |
| 16 | 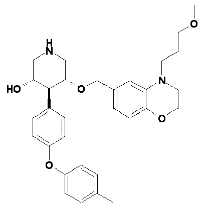 | colourless oil | 0.26 (B) | 4.25 (I) |
| 17 | 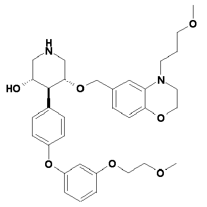 | yellow oil | 0.27 (B) | 3.92 (I) |
| 18 | 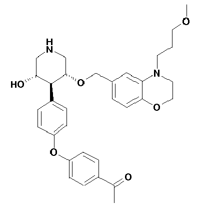 | colourless film | 0.17 (B) | 3.70 (I) |
| 19 | 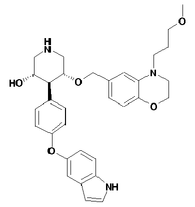 | yellowish solid | 0.11 (B) | 3.73 (I) |
| 20 | 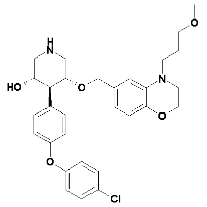 | yellow resin | 0.21 (B) | 4.32 (I) |
| 21 | 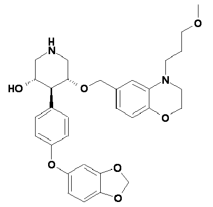 | yellowish solid | 0.26 (B) | 3.88 (I) |
| 22 | 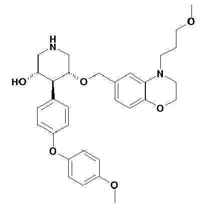 | yellowish solid | 0.21 (B) | 3.92 (I) |
| 23 | 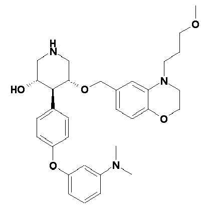 | white foam | 0.30 (B) | 3.12 (I) |
| 24 | 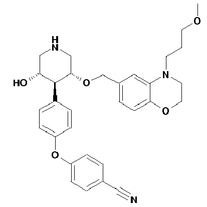 | white foam | 0.28 (B) | 3.78 (I) |
| 25 | 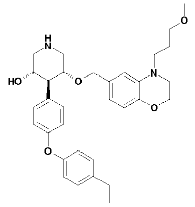 | yellowish foam | 0.36 (B) | 4.47 (I) |
| 26 | 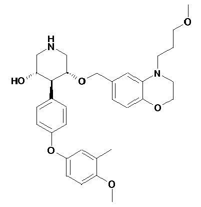 | yellowish foam | 0.30 (B) | 4.19 (I) |
| 27 | 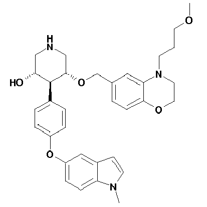 | yellowish foam | 0.25 (B) | 4.06 (I) |
| 28 | 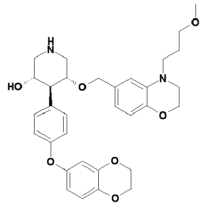 | yellowish foam | 0.23 (B) | 3.88 (I) |
| 29 | 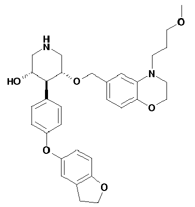 | yellowish foam | 0.33 (B) | 3.96 (I) |
| 30 | 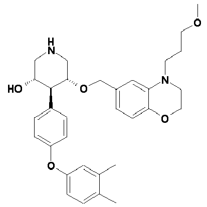 | yellowish foam | 0.33 (B) | 4.48 (I) |
| 31 | 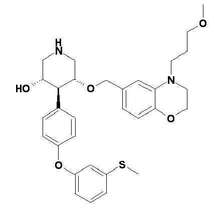 | yellowish foam | 0.27 (B) | 4.29 (I) |
| 32 | 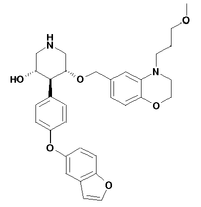 | yellowish foam | 0.25 (B) | 4.12 (I) |
| 33 | 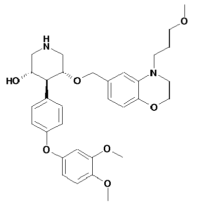 | yellow foam | 0.26 (B) | 3.73 (I) |
| 34 | 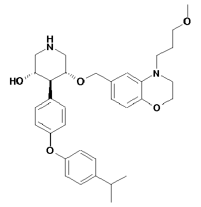 | yellow foam | 0.24 (B) | 4.77 (I) |
| 35 | 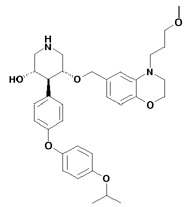 | yellowish foam | 0.24 (B) | 4.41 (I) |
| 36 | 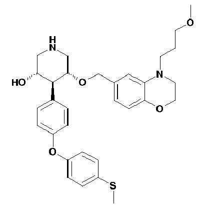 | yellow oil | 0.14 (B) | 4.29 (I) |
| 37 | 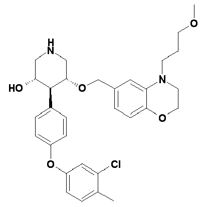 | yellow oil | 0.19 (B) | 4.65 (I) |
| 38 | 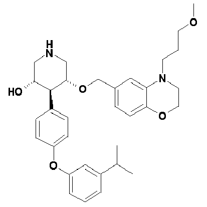 | yellow oil | 0.21 (B) | 4.73 (I) |
| 39 | 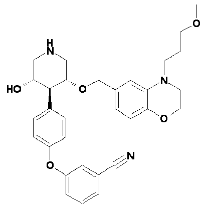 | yellowish foam | 0.31 (C) | 3.87 (I) |
| 40 | 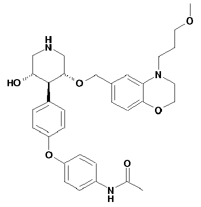 | colourless film | 0.18 (C) | 3.25 (I) |
| 41 | 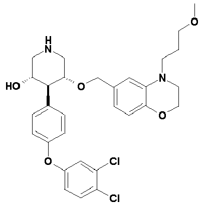 | yellowish oil | 0.38 (B) | 4.52 (I) |
| 42 | 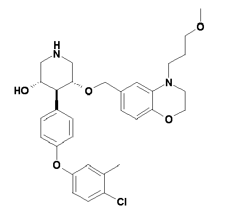 | yellowish oil | 0.26 (B) | 4.49 (I) |
| 43 | 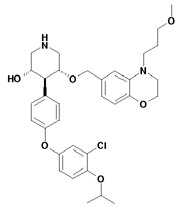 | yellowish oil | 0.26 (B) | 4.55 (I) |
| 44 | 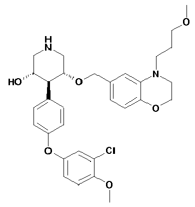 | colourless resin | 0.12 (D) | 4.21 (I) |
| 45 | 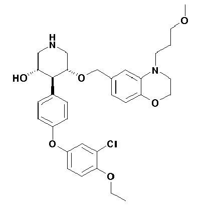 | colourless resin | 0.11 (D) | 4.51 (I) |
| 46 | 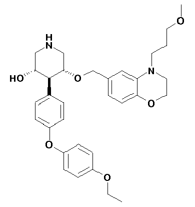 | colourless resin | 0.12 (D) | 4.25 (I) |
| 47 | 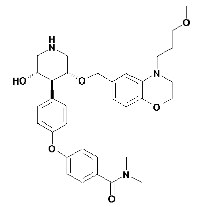 | colourless resin | 0.17 (C) | 3.29 (I) |
| 48 | 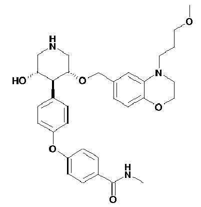 | colourless resin | 0.10 (C) | 3.11 (I) |
| 49 | 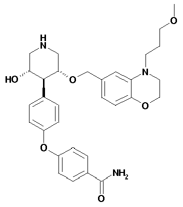 | yellow solid | 0.14 (E) | 3.05 (I) |
| 50 | 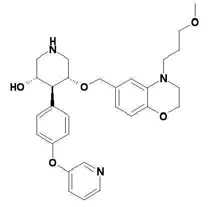 | yellowish film | 0.19 (E) | 2.81 (I) |
| 51 | 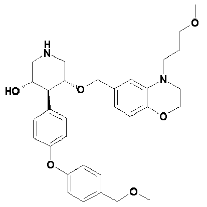 | colourless resin | 0.15 (D) | 3.91 (I) |
| 52 | 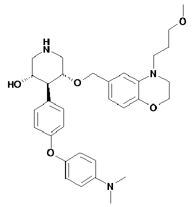 | colourless resin | 0.14 (D) | 2.91 (I) |
| 53 | 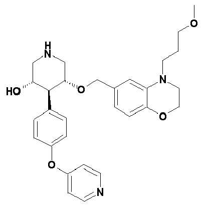 | yellow foam | 0.17 (E) | 2.71 (I) |
| 54 | 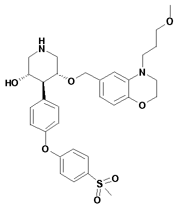 | yellow foam | 0.11 (B) | 3.46 (I) |
| 55 | 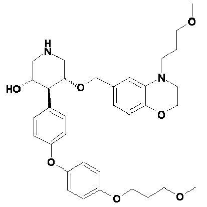 | colourless resin | 0.06 (D) | 4.12 (I) |
| 56 | 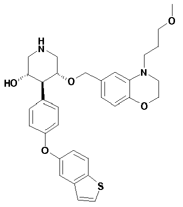 | colourless foam | 0.20 (B) | 4.36 (I) |
| 57 | 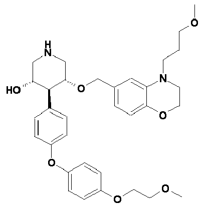 | yellowish foam | 0.19 (B) | 3.86 (I) |
| 58 | 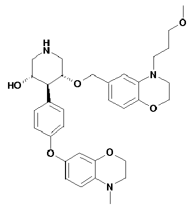 | yellowish foam | 0.16 (B) | 3.74 (I) |
| 59 | 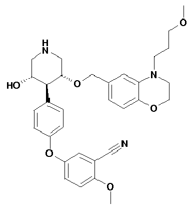 | yellowish foam | 0.16 (B) | 3.86 (I) |
| 60 | 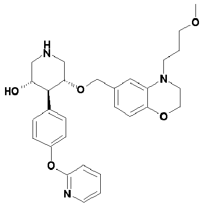 | colourless foam | 0.11 (B) | 3.49 (I) |
| 61 | 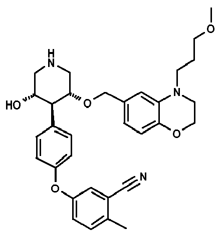 | colourless foam | 0.21 (B) | 4.04 (I) |
| 62 | 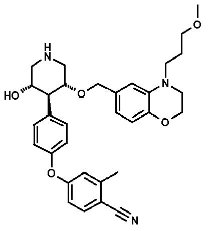 | colourless oil | 0.25 (B) | 4 (I) |
| 63 | 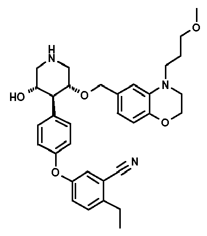 | white foam | 0.27 (B) | 4.23 (I) |
| 64 | 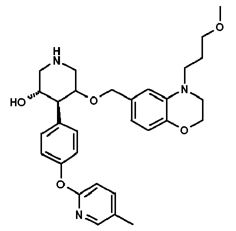 | white foam | 0.16 (B) | 3.57 (I) |
| 65 | 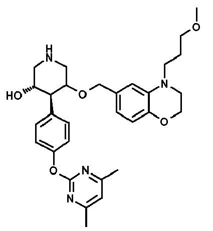 | white foam | 0.13 (B) | 3.33 (I) |
| 66 | 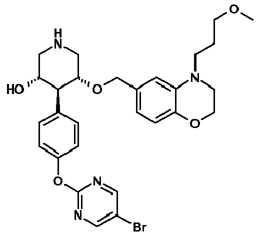 | beige wax | 0.18 (B) | 3.64 (I) |
| 67 | 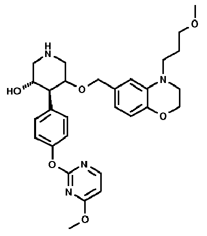 | white foam | 0.10 (B) | 3.39 (I) |
| 68 | 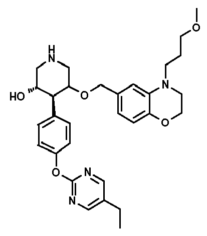 | white foam | 0.09 (B) | 3.47 (I) |
| 69 | 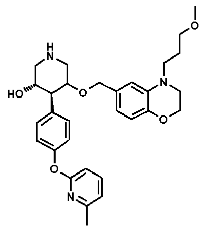 | white foam | 0.15 (B) | 3.323 (I) |
| 70 | 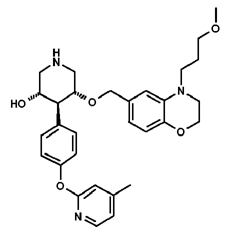 | white foam | 0.17 (B) | 3.42 (I) |
| 71 | 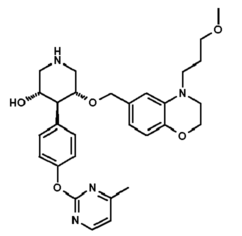 | yellowish oil | 0.31 (B) | 3.19 (I) |
| 72 | 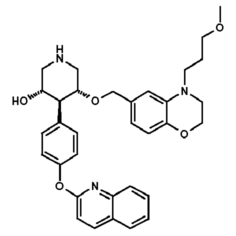 | yellowish oil | 0.30 (B) | 3.96 (I) |
| 73 | 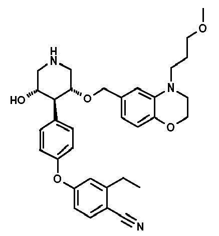 | white foam | 0.27 (B) | 4.1 (I) |
| 74 | 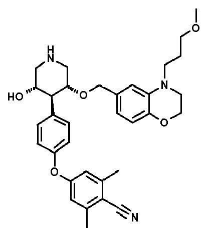 | white foam | 0.30 (B) | 4.06 (I) |
| 75 | 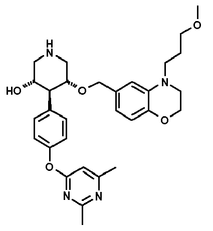 | colourless foam | 0.14 (B) | 2.799 (I) |
| 76 | 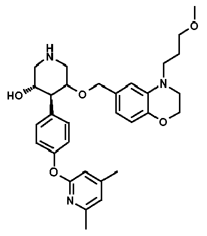 | yellowish foam | 0.27 (B) | 3.115 (I) |
| 77 | 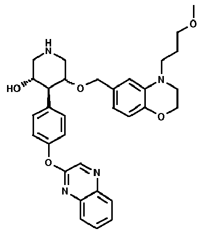 | yellowish film | 0.18 (C) | 3.768 (I) |
| 78 | 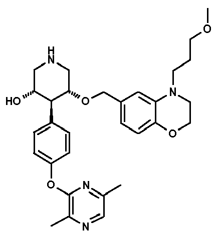 | yellowish film | 0.21 (B) | 3.456 (I) |
| 79 | 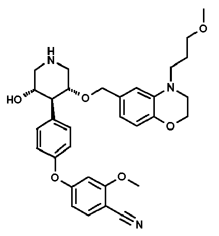 | white wax | 0.18 (B) | 3.79 (I) |
| 80 | 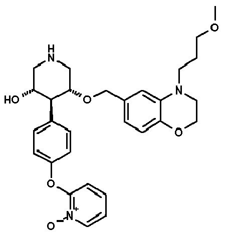 | colourless film | 0.15 (F) | 2.72 (I) |
| 81 | 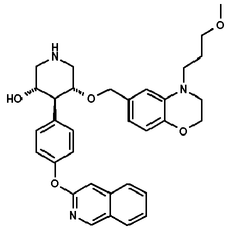 | white wax | 0.18 (B) | 3.94 (I) |
| 82 | 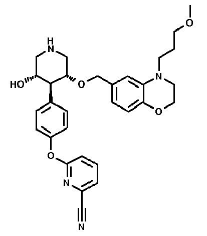 | colourless foam | 0.20 (C) | 3.59 (I) |
| 83 | 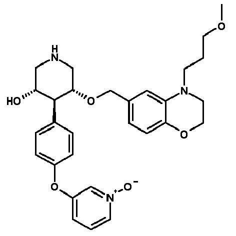 | colourless film | 0.17 (F) | 2.86 (I) |
| 84 | 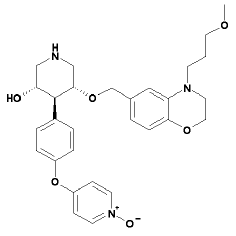 | white foam | 0.05 (B) | 2.78 (I) |
| 85 | 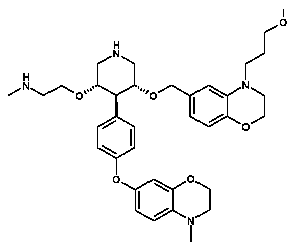 | white resin | 0.10 (G) | 3.52 (I) |

Thin-layer chromatography eluent systems:
A dichloromethane/methanol = 95:5
B dichloromethane/methanol = 9:1
C dichloromethane/methanol/25% conc. ammonia = 200:20:1
D dichloromethane/methanol/25% conc. ammonia = 200:10:1
E dichloromethane/methanol/25% conc. ammonia = 90:10:1
F dichloromethane/methanol/25% conc. ammonia = 200:40:1
G dichloromethane/methanol/25% conc. ammonia = 97:3:1

HPLC gradients on Hypersil BDS C-18 (5 µm); column: 4 x 125 mm
I 90% water*/10% acetonitrile* to 0% water*/100% acetonitrile* in 5 minutes + 2.5 minutes (1.5 ml/min)
II 95% water*/5% acetonitrile* to 0% water*/100% acetonitrile* in 40 minutes (0.8 ml/min)
* contains 0.1 % trifluoroacetic acid

The following abbreviations are used:
- M.p.: melting point (temperature)
- Rf: ratio of distance migrated by a substance to the distance of the solvent from the starting point in thin-layer chromatography
- Rt: retention time of a substance in HPLC (in minutes)

### General Method A: (N-Cbz deprotection I)

A solution of 1 mmol of "N-Cbz derivative " in 5 ml of tetrahydrofuran and 50 ml of methanol is hydrogenated in the presence of 100-200 mg Pd/C 10% at 0-20°C for 0.5 - 5 hours. The reaction mixture is clarified by filtration and the filtrate is evaporated. The title compound is obtained from the residue by flash chromatography (SiO₂ 60F).

### General Method B: (alcohol desilylation)

A solution of 1 mmol of "silyl ether" in 5 ml of tetrahydrofuran is mixed with 2.0-4.0 mmol of tetrabutylammonium fluoride (1 M solution in tetrahydrofuran), and the solution is stirred at room temperature for 1-5 hours. The reaction solution is then diluted with water and extracted twice with tert-butyl methyl ether. The combined organic phases are dried over sodium sulphate and evaporated. The title compound is obtained from the residue by flash chromatography (SiO₂ 60F).

### General Method C: (phenol arylation I)

A solution of 1 mmol of "phenol", 2-5 mmol of "boronic acid ", 2.2 mmol of copper(II) acetate, 5 mmol of triethylamine or pyridine and 700-800 mg of molecular sieves (4A, powder) in 10 ml of anhydrous dichloromethane is stirred at room temperature for 12 - 72 hours. The reaction mixture is clarified by filtration and the filtrate is evaporated. The title compound is obtained from the residue by flash chromatography (SiO₂ 60F).

### General Method D: (phenol arylation II)

A solution of 1 mmol of "phenol", 3-6 mmol of "2-heteroaryl halide" and 4-6 mmol of potassium carbonate in 4 ml of anhydrous N,N-dimethylformamide is stirred at 120°C-180°C for 1-12 hours. The reaction mixture is clarified by filtration and the filtrate is evaporated. The title compound is obained from the residue by flash chromatography (SiO₂ 60F).

### General Method E: (N-Cbz deprotection II)

A solution of 1 mmol of "N-Cbz derivative" in 30 ml of methanol, 10 ml of dioxane and 30 ml of 40% strength aqueous potassium hydroxide solution is stirred under reflux for 2-5 hours. 200 ml of water and 200 ml of ethyl acetate are added to the reaction mixture, and then the organic phase is separated off. The aqueous phase is extracted once with 200 ml of ethyl acetate, and the combined organic phases are dried with sodium sulphate and evaporated. The title compound is obtained from the residue by flash chromatography (SiO₂ 60F).

### Example 1:

### 5-[4-(3-Methoxypropyl)-3,4-dihydro-2H-benzo[1,4]oxazin-6-ylmethoxy]-4-(4-phenoxyphenyl)-piperidin-3-ol

The title compound is obtained as a colourless oil in analogy to Method A from 67.7 mg of benzyl 3-hydroxy-5-[4-(3-methoxypropyl)-3,4-dihydro-2H-benzo[1,4]oxazin-6-ylmethoxy]-4-(4-phenoxyphenyl)piperidine-1-carboxylate.

### The starting materials are prepared as follows:

### a) Benzyl 3-hydroxy-5-[4-(3-methoxypropyl)-3,4-dihydro-2H-benzo[1,4]oxazin-6-ylmethoxy]-4-(4-phenoxyphenyl)piperidine-1-carboxylate

The title compound is obtained as a colourless film in analogy to Method B from 103.4 mg of benzyl 3-[4-(3-methoxypropyl)-3,4-dihydro-2H-benzo[1,4]oxazin-6-ylmethoxy]-4-(4-phenoxyphenyl)-5-triisopropylsilanyloxypiperidine-1-carboxylate. Rf (EtOAc/heptane 1:1) = 0.20; Rt = 5.30.

### b) Benzyl 3-[4-(3-methoxypropyl)-3,4-dihydro-2H-benzo[1,4]oxazin-6-ylmethoxy]-4-(4-phenoxyphenyl)-5-triisopropylsilanyloxypiperidine-1-carboxylate

The title compound is obtained as a colourless oil in analogy to Method C from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate, 0.161 g of phenyl boronic acid, 0.104 g of copper(II) acetate and 0.18 ml of triethylamine. Rf (EtOAc/heptane 1:2) = 0.31.

### c) Benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate

A solution of 18.5 g of benzyl 4-(4-hydroxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate in 250 ml of tetrahydrofuran is mixed with 125.4 ml of borane-tetrahydrofuran complex (1 M in tetrahydrofuran) and stirred at 70°C for 1 hour (conversion checked by HPLC or DC). The reaction mixture is cooled to room temperature and, after addition of 130 ml of methanol, evaporated. The title compound is obtained as a colourless oil from the residue by flash chromatography (SiO₂ 60F). Rf (EtOAc/heptane 1:2) = 0.10.

### d) Benzyl 4-(4-hydroxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate

2.42 g of tetrakis(triphenylphosphine)palladium(0) are added to a solution of 22.6 g of benzyl 4-(4-allyloxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate in 440 ml of methanol under argon. After 5 minutes, 11.71 g of potassium carbonate are added, and the reaction mixture is stirred at room temperature for 4 hours (conversion checked by HPLC or DC). The solvent is then removed in vacuo, and the residue is mixed with 500 ml of water and extracted twice with 500 ml of tert-butyl methyl ether each time. The combined organic phases are washed once with 500 ml of water and 500 ml of brine. The organic phase is dried with sodium sulphate and evaporated. The title compound (19.59 g) is obtained as a yellow resin from the residue by flash chromatography (SiO₂ 60F). Rf (EtOAc/heptane 1:1) = 0.22.

### e) Benzyl 4-(4-allyloxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate

1.61 g of sodium hydride dispersion (60%) are added to a solution of 19.76 g of benzyl 4-(4-allyloxyphenyl)-3-hydroxy-5-triisopropylsilanyloxypiperidine-1-carboxylate and 9.95 g of 6-chloromethyl-4-(3-methoxypropyl)-4H-benz[1,4]-oxazin-3-one in 285 ml of N,N-dimethylformamide while the reaction mixture is stirred at -10°C for 1 hour and at room temperature for 18 hours. The mixture is poured into 1 M aqueous sodium bicarbonate solution (700 ml) and extracted with tert-butyl methyl ether (2 x 800 ml). The organic phases are washed successively with water (2 x 500 ml) and brine (1 x 500 ml), dried with sodium sulphate and evaporated. The title compound is obtained from the residue by flash chromatography (SiO₂ 60F). Rf (EtOAc/heptane 2:3) = 0.30; Rt = 7.15.

### f) Benzyl 4-(4-allyloxyphenyl)-3-hydroxy-5-triisopropylsilanyloxypiperidine-1-carboxylate

A mixture of 25.0 g of benzyl 3-hydroxy-4-(4-hydroxyphenyl)-5-triisopropylsilanyloxypiperidine-1-carboxylate in 350 ml of N,N-dimethylformamide is stirred with 13.9 g of potassium carbonate and 7.0 ml of allyl bromide at 70°C for 24 hours. The reaction mixture is clarified by filtration and the filtrate is evaporated. The residue is mixed with water (750 ml) and extracted with ethyl acetate (2 x 750 ml). The combined organic phases are washed with brine (1 x 750 ml), dried with sodium sulphate and evaporated. The title compound is obtained from the residue by flash chromatography (SiO₂ 60F). Rf (EtOAc/heptane 1:2) = 0.28; Rt = 6.54

### g) Benzyl 3-hydroxy-4-(4-hydroxyphenyl)-5-triisoprolpylsilanyloxypiperidine-1-carboxylate

90 ml of saturated aqueous sodium bicarbonate solution and 1.57 ml of benzyl chloroformate are added to a solution of 3.140 g of 4-(4-hydroxyphenyl)-5-triisopropylsilanyloxypiperidin-3-ol in 90 ml of ethyl acetate. The mixture is vigorously stirred for 30 minutes, and the phases are then separated. The aqueous phase is back-extracted with 100 ml of ethyl acetate, and the combined organic phases are dried over sodium sulphate and evaporated. The title compound is obtained as a colourless solid from the residue by flash chromatography (SiO₂ F60).
Rf (dichloromethane/methanol/25% conc. ammonia 200:20:1) = 0.49; Rt = 5.89.

### h) 4-(4-Hydroxyphenyl)-5-triisopropylsilanyloxypiperidin-3-ol

A solution of 113.0 g of 4-(4-benzyloxyphenyl)-1-(1-phenylethyl)-5-triisopropylsilanyloxy-piperidin-3-ol in 1.5 I of methanol is hydrogenated in the presence of 8.5 g of Pd/C 10% at room temperature for 8 hours. The reaction mixture is clarified by filtration and the filtrate is evaporated. The title compound is obtained as a colourless solid from the residue by flash chromatography (SiO₂ 60F). Rf (dichloromethane/methanol/25% conc. ammonia 200:20:1) = 0.19; Rt = 3.80.

### i) 4-(4-Benzyloxyphenyl)-1-(1-phenylethyl)-5-triisopropylsilanyloxypiperidin-3-ol

150 ml of borane-tetrahydrofuran complex (1 M in tetrahydrofuran) are added dropwise to a solution of 20.00 g of 4-(4-benzyloxyphenyl)-1-(1-phenylethyl)-3-triisopropylsilanyloxy-1,2,3,4-tetrahydropyridine in 280 ml of 1,2-dimethoxyethane at 0°C. The reaction solution is then stirred at 30°C for 3 hours. The solution is cooled to room temperature and hydrolysed with 70 ml of water. The hydrolysed solution is stirred for 5 minutes and then 56.00 g of sodium percarbonate are added, and the suspension is stirred at 50°C for 1 hour. The reaction mixture is poured into 600 ml of water and extracted with 2 x 500 ml of ethyl acetate. The combined organic phases are washed with 400 ml each of water and brine and evaporated. The title compound is obtained as a yellowish oil from the residue by flash chromatography (SiO₂ F60). Rf (EtOAc/heptane 1:2) = 0.23; Rt = 5.75.

### j) 4-(4-Benzyloxyphenyl)-1-(1-phenylethyl)-3-triisopropylsilanyloxy-1,2,3,4-tetrahydropyridine

A suspension of 14.70 g of 4-(4-benzyloxyphenyl)-1-(1-phenylethyl)-1,2,3,4-tetrahydro-pyridin-3-ol [257928-45-3] in 250 ml of dichloromethane is mixed with 6.80 ml of 2,6-lutidine and cooled to 0°C. 12.60 ml of triisopropysilyl trifluoromethanesulphonate are added dropwise, and the reaction mixture is stirred at 0°C for 1 hour. The reaction solution is poured into 400 ml of water, and the phases are separated. The aqueous phase is back-extracted with 200 ml of dichloromethane, and the combined organic phases are dried with sodium sulphate and evaporated. The title compound is obtained as a yellow-brown oil from the residue by flash chromatography (SiO₂ F60). Rf (EtOAc/heptane 1:2) = 0.66; Rt = 5.83.

### k) 6-Chloromethyl-4-(3-methoxypropyl)-4H-benz[1,4]-oxazin-3-one

A solution of 10.05 g of 6-hydroxymethyl-4-(3-methoxypropyl)-4H-benzo[1,4]oxazin-3-one in 6.40 ml of pyridine and 100 ml of dichloromethane is slowly added dropwise at 0-5°C to a precooled solution of 7.65 ml of thionyl chloride in 20 ml of dichloromethane. The reaction mixture is stirred for 1 hour each at 0°C and then at room temperature, and subsequently poured into 200 ml of ice-water. The mixture is extracted with dichloromethane (2 x 200 ml). The organic phases are washed successively with 1 M aqueous sodium bicarbonate solution (2 x 200 ml) and brine, dried with sodium sulphate and evaporated. The title compound is obtained as a colourless oil from the residue by flash chromatography (SiO₂ 60F).
Rf (EtOAc/heptane 2:1) = 0.60; Rt = 4.05.

### 1) 6-Hydroxymethyl-4-(3-methoxypropyl)-4H-benzo[1,4]oxazin-3-one

A suspension of 1.79 g of 6-hydroxymethyl-4H-benzo[1,4]oxazin-3-one, 2.20 ml of 1-chloro-3-methoxypropane, 10 g of KF on aluminium oxide and 0.033 g of potassium iodid in 150 ml of acetonitrile is stirred under reflux for 72 hours. The reaction mixture is cooled and clarified by filtration, and the filtrate is evaporated to dryness. The title compound is obtained from the residue by flash chromatography (SiO₂ 60F). Rf (CH₂Cl₂/MeOH 9:1) = 0.60; Rt = 2.74.

### m) 6-Hydroxymethyl-4H-benzo[1,4]oxazin-3-one

A mixture of 6.9 g of methyl 3-oxo-3,4-dihydro-2H-benzo[1,4]oxazine-6-carboxylate in 230 ml of tetrahydrofuran is cooled to -40°C. 88.9 ml of diisobutylaluminium hydride (1.5M in toluene) are added dropwise at -40°C over the course of 30 minutes. The reaction mixture is stirred at -40°C to -20°C for 1.5 hours and then cautiously poured into 150 ml of 2N HCl (cold). The organic phase is separated off, and the aqueous phase is extracted with tetrahydrofuran (5 x 100 ml). The organic phases are washed with brine (1 x 100 ml), filtered through cotton wool and evaporated. The title compound is obtained as beige crystals from the residue by crystallization (from ethanol). Rf = 0.16 (EtOAc/heptane 2:1); Rt = 2.23;
m.p.: 186-187°C.

The following compounds are prepared in an analogous manner to the process described in Example 1:
2 4-[4-(3-Methoxyphenoxy)phenyl]-5-[4-(3-methoxypropyl)-3,4-dihydro-2H-benzo[1,4]oxazin-6-ylmethoxy]piperidin-3-ol
3 4-[4-(3-Ethoxyphenoxy)phenyl]-5-[4-(3-methoxypropyl)-3,4-dihydro-2H-benzo[1,4]oxazin-6-ylmethoxy]piperidin-3-ol
4 4-[4-(4-Fluorophenoxy)phenyl]-5-[4-(3-methoxypropyl)-3,4-dihydro-2H-benzo[1,4]oxazin-6-ylmethoxy]piperidin-3-ol
5 4-[4-(3-Fluorophenoxy)phenyl]-5-[4-(3-methoxypropyl)-3,4-dihydro-2H-benzo[1,4]oxazin-6-ylmethoxy]piperidin-3-ol
6 5-[4-(3-Methoxypropyl)-3,4-dihydro-2H-benzo[1,4]oxazin-6-ylmethoxy]-4-[4-(3-trifluoromethylphenoxy)phenyl]piperidin-3-ol
7 5-[4-(3-Methoxypropyl)-3,4-dihydro-2H-benzo[1,4]oxazin-6-ylmethoxy]-4-[4-(3-propoxyphenoxy)phenyl]piperidin-3-ol
11 5-[4-(3-Methoxypropyl)-3,4-dihydro-2H-benzo[1,4]oxazin-6-ylmethoxy]-4-(4-o-tolyloxyphenyl)piperidin-3-ol
12 5-[4-(3-Methoxypropyl)-3,4-dihydro-2H-benzo[1,4]oxazin-6-ylmethoxy]-4-[4-(4-trifluoromethylphenoxy)phenyl]piperidin-3-ol
13 5-[4-(3-Methoxypropyl)-3,4-dihydro-2H-benzo[1,4]oxazin-6-ylmethoxy]-4-(4-m-tolyloxyphenyl)piperidin-3-ol
15 4-[4-(3-Ethylphenoxy)phenyl]-5-[4-(3-methoxypropyl)-3,4-dihydro-2H-benzo[1,4]oxazin-6-ylmethoxy]piperidin-3-ol
16 5-[4-(3-Methoxypropyl)-3,4-dihydro-2H-benzo[1,4]oxazin-6-ylmethoxy]-4-(4-p-tolyloxyphenyl)piperidin-3-ol
17 4-{4-[3-(2-Methoxyethoxy)phenoxy]phenyl}-5-[4-(3-methoxypropyl)-3,4-dihydro-2H-benzo[1,4]oxazin-6-ylmethoxy]piperidin-3-ol
18 1-[4-(4-{3-Hydroxy-5-[4-(3-methoxypropyl)-3,4-dihydro-2H-benzo[1,4]oxazin-6-lmethoxy]piperidin-4-yl}phenoxy)phenyl]ethanone
19 4-[4-(1H-Indol-5-yloxy)phenyl]-5-[4-(3-methoxypropyl)-3,4-dihydro-2H-benzo[1,4]oxazin-6-ylmethoxy]piperidin-3-ol
21 4-[4-(Benzo[1,3]dioxol-5-yloxy)phenyl]-5-[4-(3-methoxypropyl)-3,4-dihydro-2H-benzo[1,4]oxazin-6-ylmethoxy]piperidin-3-ol
22 4-[4-(4-Methoxyphenoxy)phenyl]-5-[4-(3-methoxypropyl)-3,4-dihydro-2H-benzo[1,4]oxazin-6-ylmethoxy]piperidin-3-ol
23 4-[4-(3-Dimethylaminophenoxy)phenyl]-5-[4-(3-methoxypropyl)-3,4-dihydro-2H-benzo[1,4]oxazin-6-ylmethoxy]piperidin-3-ol
24 4-(4-{3-Hydroxy-5-[4-(3-methoxypropyl)-3,4-dihydro-2H-benzo[1,4]oxazin-6-ylmethoxyl]poperidin-4-yl}phenoxy)benzonitrile
25 4-[4-(4-Ethylphenoxy)phenyl]-5-[4-(3-methoxypropyl)-3,4-dihydro-2H-benzo[1,4]oxazin-6-ylmethoxy]piperidin-3-ol
26 4-[4-(4-Methoxy-3-methylphenoxy)phenyl]-5-[4-(3-methoxypropyl)-3,4*-*dihydro-2H-benzo[1,4]oxazin-6-ylmethoxy]piperidin-3-ol
27 5-[4-(3-Methoxypropyl)-3,4-dihydro-2H-benzo[1,4]oxazin-6-ylmethoxy]-4-[4-(1-methyl-1 H-indol-5-yloxy)phenyl]piperidin-3-ol
28 4-[4-(2,3-Dihydrobenzo[1,4]dioxin-6-yloxy)phenyl]-5-[4-(3-methoxypropyl)-3,4-dihydro-2H-benzor[1,4]oxazin-6-ylmethoxy]piperidin-3-ol
29 -[4-(2,3-Dihydrobenzofuran-5-yloxy)phenyl]-5-[4-(3-methoxypropyl)-3,4-dihydro-2H-benzo[1,4]oxazin-6-ylmethoxy]piperidin-3-ol
30 4-[4-(3,4-Dimethylphenoxy)phenyl]-5-[4-(3-methoxypropyl)-3,4-dihydro-2H-benzo[1,4]oxazin-6-ylmethoxy]piperidin-3-ol
32 4-[4-(Benzofuran-5-yloxy)phenyl]-5-[4-(3-methoxypropyl)-3,4-dihydro-2H-benzo[1,4]oxazin-6-ylmethoxy]piperidin-3-ol
33 4-[4-(3,4-Dimethoxyphenoxy)phenyl]-5-[4-(3-methoxypropyl)-3,4-dihydro-2H-benzo[1,4]oxazin-6-ylmethoxy]piperidin-3-ol
34 4-[4-(4-Isopropylphenoxy)phenyl]-5-[4-(3-methoxypropyl)-3,4-dihydro-2H-benzo[1,4]oxazin-6-ylmethoxy]piperidin-3-ol
35 4-[4-(4-Isopropoxyphenoxy)phenyl]-5-[4-(3-methoxypropyl)-3,4-dihydro-2H -benzo[1,4]oxazin-6-ylmethoxy]piperidin-3-ol
38 4-[4-(3-Isopropylphenoxy)phenyl]-5-[4-(3-methoxypropyl)-3,4-dihydro-2H-benzo[1,4]oxazin-6-ylmethoxy]piperidin-3-ol
39 3-(4-{3-Hydroxy-5-[4-(3-methoxypropyl)-3,4-dihydro-2H-benzo[1,4]oxazin-6-ylmethoxy]piperidin-4-yl}phenoxy)benzonitrile
40 N-[4-(4-{3-Hydroxy-5-[4-(3-methoxypropyl)-3,4-dihydro-2H-benzo[1,4]oxazin-6-ylmethoxy]piperidin-4-yl}phenoxy)phenyl]acetamide
46 4-[4-(4-Ethoxyphenoxy)phenyl]-5-[4-(3-methoxypropyl)-3,4-dihydro-2H-benzo[1,4]oxazin-6-ylmethoxy]piperidin-3-ol
47 4-(4-{3-Hydroxy-5-[4-(3-methoxypropyl)-3,4-dihydro-2H-benzo[1,4]oxazin-6-ylmethoxy]piperidin-4-yl}phenoxy)-N,N-dimethylbenzamide
48 4-(4-{3-Hydroxy-5-[4-(3-methoxypropyl)-3,4-dihydro-2H-benzo[1,4]oxazin-6-ylmethoxy]piperidin-4-yl}phenoxy)-N-methylbenzamide
49 4-(4-{3-Hydroxy-5-[4-(3-methoxypropyl)-3,4-dihydro-2H-benzo[1,4]oxazin-6-ylmethoxy]piperidin-4-yl}phenoxy)benzamide
50 5-[4-(3-Methoxypropyl)-3,4-dihydro-2H-benzo[1,4]oxazin-6-ylmethoxy]-4-[4-(pyridin-3-yloxy)phenyl]piperidin-3-ol
51 4-[4-(4-Methoxymethylphenoxy)phenyl]-5-[4-(3-methoxypropyl)-3,4-dihydro-2H-benzo[1,4]oxazin-6-ylmethoxy]piperidin-3-ol
52 4-[4-(4-Dimethylaminophenoxy)phenyl]-5-[4-(3-methoxypropyl)-3,4-dihydro-2H-benzo[1,4]oxazin-6-ylmethoxy]piperidin-3-ol
53 5-[4-(3-Methoxypropyl)-3,4-dihydro-2H-benzo[1,4]oxazin-6-ylmethoxy]-4-[4-(pyridin-4-yloxy)phenyl]piperidin-3-ol
54 4-[4-(4-Methanesulphonylphenoxy)phenyl]-5-[4-(3-methoxypropyl)-3,4-dihydro-2H-benzo[1,4]oxazin-6-ylmethoxy]piperidin-3-ol
55 4-{4-[4-(3-Methoxypropoxy)phenoxy]phenyl}-5-[4-(3-methoxypropyl)-3,4-dihydro-2H-benzo[1,4]oxazin-6-ylmethoxy]piperidin-3-ol
57 4-{4-[4-(2-Methoxyethoxy)phenoxylphenyl}-5-[4-(3-methoxypropyl)-3,4-dihydro-2H-benzo[1,4]oxazin-6-ylmethoxy]piperidin-3-ol
58 5-[4-(3-Methoxypropyl)-3,4-dihydro-2H-benzo[1,4]oxazin-6-ylmethoxy]-4-[4-(4-methyl-3,4-dihydro-2H-benzo[1,4]oxazin-7-yloxy)phenyl]piperidin-3-ol
79 4-(4-{3-Hydroxy-5-[4-(3-methoxypropyl)-3,4-dihydro-2H-benzo[1,4]oxazin-6-ylmethoxy]piperidin-4-yl}phenoxy)-2-methoxybenzonitrile

### Example 9:

### 4-{4-[4-(1-Hydroxy-1-methylethyl)phenoxy]phenyl}-5-[4-(3-methoxypropyl)-3,4-dihydro-2H-benzo[1,4]oxazin-6-ylmethoxy]piperidin-3-ol

The title compound is obtained as a colourless film in analogy to Method A from 22.6 mg of benzyl 3-hydroxy-4-{4-[4-(1-hydroxy-1-methylethyl)phenoxy]phenyl}-5-[4-(3-methoxypropyl)-3,4-dihydro-2H-benzo[1,4]oxazin-6-ylmethoxy]piperidine-1-carboxylate.

The starting materials are prepared as follows:

### a) Benzyl 3-hydroxy-4-{4-[4-(1-hydroxy-1-methylethyl)phenoxy]phenyl}-5-[4-(3-methoxypropyl)-3,4-dihydro-2H-benzo[1,4]oxazin-6-ylmethoxy]piperidine-1-carboxylate

0.06 ml of methylmagnesium bromide solution (3M solution in diethyl ether) is added dropwise to a solution of 49 mg of benzyl 4-[4-(4-acetylphenoxy)phenyl]-3-hydroxy-5-[4-(3-methoxypropyl)-3,4-dihydro-2H-benzo[1,4]oxazin-6-ylmethoxy]piperidine-1-carboxylate in 0.3 ml of tetrahydrofuran under argon at room temperature and stirred for 2 hours. 3 ml of 0.5M aqueous HCl solution are added to the reaction mixture, and it is extracted with 15 ml of ethyl acetate. The organic phase is separated off, dried with sodium sulphate and evaporated. The title compound is obtained from the residue by flash chromatography (SiO₂ 60F). Rf (EtOAc/heptane 3:1) = 0.22; Rt = 4.84.

### b) Benzyl 4-[4-(4-acetylphenoxy)phenyl]-3-hydroxy-5-[4-(3-methoxypropyl)-3,4-dihydro-2H-benzo[1,4]oxazin-6-ylmethoxy]piperidine-1-carboxylate

The title compound is obtained as a colourless film in analogy to Method B from 187.3 mg of benzyl 4-[4-(4-acetylphenoxy)phenyl]-3-[4-(3-methoxypropyl)-3,4-dihydro-2H-benzo[1,4]oxazin-6-ylmethoxy]-5-triisopropylsilanyloxypiperidine-1-carboxylate.
Rf (EtOAc/heptane 3:1) = 0.24; Rt = 4.97.

### c) Benzyl 4-[4-(4-acetylphenoxy)phenyl]-3-[4-(3-methoxypropyl)-3,4-dihydro-2H-benzo[1,4]oxazin-6-ylmethoxy]-5-triisopropylsilanyloxypiperidine-1-carboxylate

The title compound is obtained as a colourless oil in analogy to Method C from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1 c), 0.235 g of 4-acetylphenylboronic acid, 0.113 g of copper(II) acetate and 0.19 ml of triethylamine.
Rf (EtOAc/heptane 1:2) = 0.16.

The following compound is prepared in an analogous manner to the process described in Example 9:

### 10 4-{4-[4-(1-Methoxv-1-methylethyl)phenoxy]phenyl}-5-[4-(3-methoxypropyl)-3,4-dihydro-2H-benzo[1,4]oxazin-6-ylmethoxy]piperidin-3-ol

### Example 14:

### 4-{4-[3-(3-Methoxypropoxy)phenoxy]phenyl}-5-[4-(3-methoxypropyl)-3,4-dihydro-2H-benzo[1,4]oxazin-6-ylmethoxy]piperidin-3-ol

The title compound is obtained as a colourless oil in analogy to Example 1 in 3 steps (Methods A, B and C) from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1c), 0.3254 g of 3-(3-methoxypropoxy)phenylboronic acid, 0.1134 g of copper(II) acetate and 0.19 ml of triethylamine.

The starting materials are prepared as follows:

### a) 3-(3-Methoxypropoxy)phenylboronic acid

6.71 ml of n-butyllithium solution (1.6M in hexane) are added dropwise to a solution of 2.5 g of 1-bromo-3-(3-methoxypropoxy)benzene in 14 ml of dry tetrahydrofuran at -78°C. After stirring at this temperature for 15 minutes, 2.78 ml of triisopropyl borate are added, and the mixture is then slowly warmed to room temperature. 20 ml of 1 N HCl and 5 ml of conc. HCl are added to the reaction mixture, and the organic solvents are evaporated off in vacuo. The precipitate which has separated out is filtered off with suction and washed twice with ice-water. Drying under high vacuum affords 0.987 g of the title compound as a yellowish brown solid. Rt = 3.04.

### b) 1-Bromo-3-(3-methoxypropoxy)benzene

A mixture of 5.0 g of 3-bromophenol, 7.99 g of potassium carbonate and 3.77 g of 1-chloro-3-methoxypropane in 90 ml of dimethylformamide is stirred at 100°C for 4.5 hours. The reaction mixture is clarified by filtration and the filtrate is evaporated in vacuo. The residue is taken up in 100 ml of tert-butyl methyl ether, and the organic phase is washed once each with 50 ml of 1 N NaOH, 50 ml of water and 50 ml of brine. The organic phase is dried with sodium sulphate, filtered and evaporated. 7.08 g of the title compound are obtained as an orange oil. Rt = 4.91.

### Example 59:

### 5-(4-{3-Hydroxy-5-[4-(3-methoxypropyl)-3,4-dihydro-2H-benzo[1,4]oxazin-6-ylmethoxy]-piperidin-4-yl}phenoxy)-2-methoxybenzonitrile

The title compound is obtained as a yellowish foam in analogy to Example 1 in 3 steps (Methods A, B and C) from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1c), 0.2604 g of 3-cyano-4-methoxyphenylboronic acid, 0.1134 g of copper(II) acetate and 0.19 ml of triethylamine.

The starting material is prepared as follows:

### a) 3-Cyano-4-methoxyphenylboronic acid

16.23 ml of n-butyllithium solution (1.6M in hexane) are added dropwise to a solution of 5.00 g of 5-bromo-2-methoxybenzonitrile in 90 ml of dry tetrahydrofuran at -78°C. After stirring at this temperature for 45 minutes, 6.71 ml of triisopropylborate are added, and the mixture is then slowly warmed to -20°C. 50 ml of 1 N HCl are added to the reaction mixture. The phase is separated and the aqueous phase is extracted three more times with 100 ml of diethyl ether each time. The combined organic phases are dried with sodium sulphate, filtered and evaporated. The remaining oil is mixed with pentane, and the precipitate which separates out is filtered off with suction and washed once with a little dichloromethane. Drying under high vacuum affords the title compound as a white solid. Rt = 2.74.

### Example 61:

### 5-(4-{3-Hydroxy-5-[4-(3-methoxypropyl)-3,4-dihydro-2H-benzo[1,4]oxazin-6-ylmethoxy]-piperidin-4-yl}phenoxy)-2-methylbenzonitrile

The title compound is obtained as a yellow foam in analogy to Example 1 in 3 steps (Methods A, B and C) from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1c), 0.2293 g of 3-cyano-4-methylphenylboronic acid, 0.1134g of copper(II) acetate and 0.19 ml of triethylamine.

The starting material is prepared as follows:

### a) 3-Cyano-4-methylphenylboronic acid

17.5 ml of n-butyllithium solution (1.6M in hexane) are added dropwise to a solution of 5 g of 5-bromo-2-methylbenzonitrile in 80 ml of dry tetrahydrofuran at -78°C. After stirring at this temperature for 35 minutes, 7.3 ml of triisopropyl borate are added. After one hour, 50 ml of 1 N HCl are added to the reaction mixture at -78°C, and the mixture is warmed to room temperature. The phases are separated and the aqueous phase is extracted three more times with 100 ml of diethyl ether each time. The combined organic phases are dried with sodium sulphate, and the solvent is concentrated to 5 ml. The residue is mixed with 150 ml of pentane. The precipitate which has separated out is filtered off with suction and washed twice with pentane. Drying under high vacuum results in 2.85 g of the title compond as a pale yellow solid. Rt = 2.98.

### Example 62:

### 4-(4-{3-Hydroxy-5-[4-(3-methoxypropyl)-3,4-dihydro-2H-benzo[1,4]oxazin-6-ylmethoxy]-piperidin-4-yl}phenoxy)-2-methylbenzonitrile

The title compound is obtained as a colourless oil in analogy to Example 1 in 3 steps (Methods A, B and C) from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1c), 0.0923g of 4-cyano-3-methylphenylboronic acid, 0.1134g of copper(II) acetate and 0.12 ml of pyridine.

The starting material is prepared as follows:

### a) 4-Cyano-3-methylphenylboronic acid

10.21 ml of n-butyllithium solution (1.6M in hexane) are added dropwise to a solution of 3.00 g of 4-bromo-2-methylbenzonitrile in 50 ml of dry tetrahydrofuran at -78°C. After stirring at this temperature for 30 minutes, 4.22 ml of triisopropyl borate are added, and then the mixture is stirred at -78°C for a further 45 minutes. 30 ml of 1 N HCl are added to the reaction mixture. The phases are separated and the aqueous phase is extracted three more times with 100 ml of diethyl ether/tetrahydrofuran (1:1) each time. The combined organic phases are dried with sodium sulphate, filtered and concentrated to 4 ml in vacuo. The remaining oil is mixed with 100 ml of pentane, and the precipitate which has separated out is filtered off with suction and washed twice with pentane. Drying under high vacuum results in 1.82 g of the title compound as a white solid. Rt = 2.93.

### Example 63:

### 2-Ethyl-5-(4-{3-hydroxy-5-[4-(3-methoxypropyl)-3,4-dihydro-2H-benzo[1,4]oxazin-6-ylmethoxy]piperidin-4-yl}phenoxy)benzonitrile

The title compound is obtained as a white foam in analogy to Example 1 in 3 steps (Methods A, B and C) from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1c), 0.1003 g of 3-cyano-4-ethylphenylboronic acid, 0.1134 g of copper(II) acetate and 0.12 ml of pyridine.

The starting materials are prepared as follows:

### a) 3-Cyano-4-ethylphenylboronic acid

9.13 ml of n-butyllithium solution (1.6M in hexane) are added dropwise to a solution of 3.00 g of 5-bromo-2-ethylbenzonitrile in 50 ml of dry tetrahydrofuran at -78°C. After stirring at this temperature for 20 minutes, 3.78 ml of triisopropyl borate are added dropwise. The reaction mixture is stirred at -78°C for 45 minutes and then 30 ml of 1 N HCl are added. After warming to room temperature, the phases are separated and the aqueous phase is extracted three more times with 100 ml of diethyl ether/tetrahydrofuran (1:1) each time. The combined organic phases are dried with sodium sulphate, filtered and concentrated to 4 ml in vacuo. The remaining oil is mixed with 100 ml of pentane, and the precipitate which has separated out is filtered off with suction and washed twice with a little pentane. Drying under high vacuum results in 1.27 g of the title compound as a beige solid. Rt = 3.34.

### b) 5-Bromo-2-ethylbenzonitrile

9.74 ml of 2-ethylbenzonitrile are added dropwise to 100 g of Na-X zeolite (Linde 13X, STREM) with vigorous stirring. After the exothermic reaction, 5.6 ml of bromine are added dropwise at 55°C, and vigorous stirring of the mixture is continued. The temperature is kept at 45-50°C by means of an ice bath. After the addition is complete, the ice bath is removed, and the mixture is stirred at 50°C for 20 hours. 300 ml of methanol are added to the reaction mixture while stirring, and the suspension is clarified by filtration through Hyflow. The filtrate is evaporated and the residue is partitioned between 100 ml of ethyl acetate and 50 ml of saturated sodium bicarbonate solution. The organic phase is dried with sodium sulphate, filtered and evaporated. The title compound is obtained as a beige crystalline solid from the residue by flash chromatography. Rt = 4.87.

### Example 73:

### 2-Ethyl-4-(4-{3-hydroxy-5-[4-(3-methoxypropyl)-3,4-dihydro-2H-benzo[1,4]oxazin-6-ylmethoxy]piperidin-4-yl}phenoxy)benzonitrile

The title compound is obtained as a white foam in analogy to Example 1 in 3 steps (Methods A, B and C) from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1c), 0.1071 g of 4-cyano-3-ethylphenylboronic acid, 0.1134 g of copper(II) acetate and 0.12 ml of pyridine.

The starting material is prepared as follows:

### a) 4-Cyano-3-ethylphenylboronic acid

3.66 ml of n-butyllithium solution (1.6M in hexane) are added dropwise to a solution of 1.14 g of 4-bromo-2-ethylbenzonitrile in 20 ml of dry tetrahydrofuran at -78°C. After stirring at this temperature for 20 minutes, 1.51 ml of triisopropyl borate are added. After 45 minutes, 12 ml of 1 N HCl are added to the reaction mixture, which is warmed to room temperature. The phases are separated and the aqueous phase is extracted three more times with 100 ml of diethyl ether/tetrahydrofuran (1:1) each time. The combined organic phases are dried with sodium sulphate, filtered and concentrated to 4 ml in vacuo. The remainig oil is mixed with 40 ml of pentane, and the precipitate which has separated out is filtered off with suction and washed twice with pentane. Drying under high vacuum results in the title compound as a yellow solid. Rt = 3.27.

### Example 74:

### 4-(4-{3-Hydroxy-5-[4-(3-methoxypropyl)-3,4-dihydro-2H-benzo[1,4]oxazin-6-ylmethoxy]-piperidin-4-yl}phenoxy)-2,6-dimethylbenzonitrile

The title compound is obtained as a white foam in analogy to Example 1 in 3 steps (Methods A, B and C) from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1c), 0.1172 g of 4-cyano-3,5-dimethylphenylboronic acid, 0.1134 g of copper(II) acetate and 0.12 ml of pyridine.

The starting material is prepared as follows:

### a) 4-Cyano-3,5-dimethylphenylboronic acid

4.55 ml of n-butyllithium solution (1.6M in hexane) are added dropwise to a solution of 1.39 g of 4-bromo-2,6-dimethylbenzonitrile in 25 ml of dry tetrahydrofuran at -78°C. After stirring at this temperature for 20 minutes, 1.88 ml of triisopropyl borate are added. After 45 minutes, 15 ml of 1 N aqueous HCl are added to the reaction mixture, which is warmed to room temperature. The phases are separated and the aqueous phase is extracted three more times with 100 ml of diethyl ether/tetrahydrofuran (1:1) each time. The combined organic phases are dried with sodium sulphate, filtered and concentrated to 4 ml in vacuo. The remaining oil is mixed with 50 ml of pentane, and the precipitate which has separated out is filtered off with suction and washed twice with pentane. Drying under high vacuum results in the title compound as a beige solid. Rt = 3.20.

### Example 8:

### 4-[4-(3-Chlorophenoxy)phenyl]-5-[4-(3-methoxypropyl)-3,4-dihydro-2H-benzo[1,4]oxazin-6-ylmethoxy]piperidin-3-ol

The title compound is obtained as a colourless film in analogy to Method E from 93.9 mg of benzyl 4-[4-(3-chlorophenoxy)phenyl]-3-hydroxy-5-[4-(3-methoxypropyl)-3,4-dihydro-2H-benzo[1,4]oxazin-6-ylmethoxy]piperidine-1-carboxylate.

The starting materials are prepared as follows:

### a) Benzyl 4-[4-(3-chlorophenoxy)phenyl]-3-hydroxy-5-[4-(3-methoxypropyl)-3,4-dihydro-2H-benzo[1,4]oxazin-6-ylmethoxy]piperidine-1-carboxylate

The title compound is obtained as a colourless oil in analogy to Method B from 131.7 mg of benzyl 4-[4-(3-chlorophenoxy)phenyl]-3-[4-(3-methoxypropyl)-3,4-dihydro-2H-benzo[1,4]oxazin-6-ylmethoxy]-5-triisopropylsilanyloxypiperidine-1-carboxylate.
Rf (EtOAc/heptane 1:1) = 0.17; Rt = 5.55.

### b) Benzyl 4-[4-(3-chlorophenoxy)phenyl]-3-[4-(3-methoxypropyl)-3,4-dihydro-2H-benzo-[1,4]oxazin-6-ylmethoxy]-5-triisopropylsilanyloxypiperidine-1-carboxylate

The title compound is obtained as a yellow oil in analogy to Method C from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1c), 0.2241 g of 3-chlorophenyl-boronic acid, 0.113 g of copper(II) acetate and 0.19 ml of triethylamine. Rf (EtOAc/heptane 1:2) = 0.26.

The following compounds are prepared in an analogous manner to the process described in Example 8:
20 4-[4-(4-Chlorophenoxy)phenyl]-5-[4-(3-methoxypropyl)-3,4-dihydro-2H-benzo[1,4]oxazin-6-ylmethoxy]piperidin-3-ol
31 5-[4-(3-Methoxypropyl)-3,4-dihydro-2H-benzo[1,4]oxazin-6-ylmethoxy]-4-[4-(3-methylsulphanylphenoxy)phenyl]piperidin-3-ol
36 5-[4-(3-Methoxypropyl)-3,4-dihydro-2H-benzo[1,4]oxazin-6-ylmethoxy]-4-[4-(4-methyl-sulphanylphenoxy)phenyl]piperidin-3-ol
37 4-[4-(3-Chloro-4-methylphenoxy)phenyl]-5-[4-(3-methoxypropyl)-3,4-dihydro-2H-benzo[1,4]oxazin-6-ylmethoxy]piperidin-3-ol
41 4-[4-(3,4-Dichlorophenoxy)phenyl]-5-[4-(3-methoxypropyl)-3,4-dihydro-2H-benzo-[1,4]oxazin-6-ylmethoxy]piperidin-3-ol
42 4-[4-(4-Chloro-3-methylphenoxy)phenyl]-5-[4-(3-methoxypropyl)-3,4-dihydro-2H-benzo[1,4]oxazin-6-ylmethoxy]piperidin-3-ol
43 4-[4-(3-Chloro-4-isopropoxyphenoxy)phenyl]-5-[4-(3-methoxypropyl)-3,4-dihydro-2H-benzo[1,4]oxazin-6-ylmethoxy]piperidin-3-ol
44 4-[4-(3-Chloro-4-methoxyphenoxy)phenyl]-5-[4-(3-methoxypropyl)-[3,4-dihydro-2H-benzo[1,4]oxazin-6-ylmethoxy]piperidin-3-ol
45 4-[4-(3-Chloro-4-ethoxyphenoxy)phenyl]-5-[4-(3-methoxypropyl)-3,4-dihydro-2H-benzo[1,4]oxazin-6-ylmethoxy]piperidin-3-ol

### Example 56:

### 4-[4-(Benzo[b]thiophen-5-yloxy)phenyl]-5-[4-(3-methoxypropyl)-3,4-dihydro-2H-benzo[1,4]oxazin-6-ylmethoxyl]piperidin-3-ol

The title compound is obtained as a colourless foam in analogy to Example 8 in 3 steps (Methods C, B and E) from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1c), 0.2502 g of benzo[b]thiophene-5-boronic acid. 0.1134 g of copper(II) acetate and 0.19 ml of triethylamine.

The starting material is prepared as follows:

### a) Benzo[b]thiophene-5-boronic acid

A solution of 0.6 g of 2-(1-benzothiophen-5-yl)-4,4,5,5-tetramethyl-1,3,2-dioxaborolane in 2.5 ml of dichloromethane is added dropwise to a solution of boron trichloride in dichloromethane (1 M, 14.3 ml) at -78°C. After 15 minutes, the reaction mixture is warmed to room temperature, and 8 ml of methanol are added dropwise. It is then evaporated to dryness and dried under high vacuum overnight. 0.350 g of the title compound is obtained as a grey powder. Rt = 3.33.

### Example 60:

### 5-[4-(3-Methoxypropyl)-3,4-dihydro-2H-benzo[1,4]oxazin-6-ylmethoxyl-4-[4-(pyridin-2-yloxy)-phenyl]piperidin-3-ol

The title compound is obtained as a colourless oil in analogy to Method A from 68.8 mg of benzyl 3-hydroxy-5-[4-(3-methoxypropyl)-3,4-dihydro-2H-benzo[1,4]oxazin-6-ylmethoxy]-4-[4-(pyridin-2-yloxy)phenyl]piperidine-1-carboxylate.

The starting materials are prepared as follows:

### a) Benzyl 3-hydroxy-5-[4-(3-methoxypropyl)-3,4-dihydro-2H-benzo[1,4]oxazin-6-ylmethoxy]-4-[4-(pyridin-2-yloxy)phenyl]piperidine-1-carboxylate

The title compound is obtained as a colourless film in analogy to Method B from 180 mg of benzyl 3-[4-(3-methoxypropyl)-3,4-dihydro-2H-benzo[1,4]oxazin-6-ylmethoxy]-4-[4-(pyridin-2-yloxy)phenyl]-5-triisopropylsilanyloxypiperidine-1-carboxylate. Rf (EtOAc/heptane 3:1) = 0.12; Rt = 4.76.

### b) Benzyl 3-[4-(3-methoxypropyl)-3,4-dihydro-2H-benzo[1,4]oxazin-6-ylmethoxy]-4-[4-(pyridin-2-yloxy)phenyl]-5-triisopropylsilanyloxypiperidine-1-carboxylate

The title compound is obtained as a yellowish oil in analogy to Method D from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1c), 0.133 g of 2-bromopyridine and 0.157 g of potassium carbonate. Rf (EtOAc/heptane 2:3) = 0.20.

The following compounds are prepared in an analogous manner to the process described in Example 60:
64 5-[4-(3-Methoxypropyl)-3,4-dihydro-2H-benzo[1,4]oxazin-6-ylmethoxy]-4-[4-(5-methyl-pyridin-2-yloxy)phenyl]piperidin-3-ol
65 4-[4-(4,6-Dimethylpyrimidin-2-yloxy)phenyl-5-[4-(3-methoxypropyl)-3,4-dihydro-2H-benzo[1,4]oxazin-6-ylmethoxy]piperidin-3-ol
67 5-[4-(3-Methoxypropyl)-3,4-dihydro-2H-benzo[1,4]oxazin-6-ylmethoxy]-4-[4-(4-methoxypyrimidin-2-yloxy)phenyl]piperidin-3-ol
68 4-[4-(5-Ethylpyrimidin-2-yloxy)phenyl]-5-[4-(3-methoxypropyl)-3,4-dihydro-2H-benzo[1,4]oxazin-6-ylmethoxy]piperidin-3-ol
69 5-[4-(3-Methoxypropyl)-3,4-dihydro-2H-benzo[1,4]oxazin-6-ylmethoxy]-4-[4-(6-methyl-pyridin-2-yloxy)phenyl]piperidin-3-ol
70 5-[4-(3-Methoxypropyl)-3,4-dihydro-2H-benzo[1.4]oxazin-6-ylmethoxy]-4-[4-(4-methyl-pyridin-2-yloxy)phenyl]piperidin-3-ol
71 5-[4-(3-Methoxypropyl)-3,4-dihydro-2H-benzo[1,4]oxazin-6-ylmethoxy]-4-[4-(4-methyl-pyrimidin-2-yloxy)phenyl]piperidin-3-ol
72 5-[4-(3-Methoxypropyl)-3,4-dihydro-2H-benzo[1,4]oxazin-6-ylmethoxy]-4-[4-(quinolin-2-yloxy)phenyl]piperidin-3-ol
75 4-[4-(2,6-Dimethylpyrimidin-4-yloxy)phenyl]-5-[4-(3-methoxypropyl)-3,4-dihydro-2H-benzo[1,4]oxazin-6-ylmethoxy]piperidin-3-ol
76 4-[4-(4,6-Dimethylpyridin-2-yloxy)phenyl]-5-[4-(3-methoxypropyl)-3,4-dihydro-2H-benzo[1,4]oxazin-6-ylmethoxy]piperidin-3-ol
77 5-[4-(3-Methoxypropyl)-3,4-dihydro-2H-benzo[1,4]oxazin-6-ylmethoxy]-4-[4-(quinoxalin-2-yloxy)phenyl]piperidin-3-ol
78 4-[4-(3,6-Dimethylpyrazin-2-yloxy)phenyl]-5-[4-(3-methoxypropyl)-3,4-dihydro-2H-benzo[1,4]oxazin-6-ylmethoxy]piperidin-3-ol
81 4-[4-(Isoquinolin-3-yloxy)phenyl]-5-[4-(3-methoxypropyl)-3,4-dihydro-2H-benzo[1,4]oxazin-6-ylmethoxy]piperidin-3-ol
82 6-(4-{3-Hydroxy-5-[4-(3-methoxypropyl)-3,4-dihydro-2H-benzo[1,4]oxazin-6-ylmethoxyl]piperidin-4-yl}phenoxy)pyridine-2-carbonitrile

### Example 66:

### 4-[4-(5-Bromopyrimidin-2-yloxy)phenyl]-5-[4-(3-methoxypropyl)-3,4-dihydro-2H-benzo[1,4]oxazin-6-ylmethoxy]piperidin-3-ol

A solution of 139.8 mg of benzyl 4-[4-(5-bromopyrimidin-2-yloxy)phenyl]-3-hydroxy-5-[4-(3-methoxypropyl)-3,4-dihydro-2H-benzo[1,4]oxazin-6-ylmethoxy]piperidine-1-carboxylate in 0.5 ml of dichloromethane is added to a mixture of 0.232 ml of boron trifluoride etherate and 0.4 ml of ethanethiol. The reaction mixture is stirred at room temperature under argon for 20 hours. The solvent is then evaporated off, and 2 ml of water are added to the residue. The aqueous phase is extracted three times with 5 ml of ethyl acetate each time. The combined organic phases are dried with sodium sulphate, filtered and evaporated. The title compound is obtained as a beige wax from the residue by flash chromatography (SiO₂ 60F).

The starting materials are prepared as follows:

### a) Benzyl 4-[4-(5-bromopyrimidin-2-yloxy)phenyl]-3-hydroxy-5-[4-(3-methoxypropyl)-3,4-dihydro-2H-benzo[1,4]oxazin-6-ylmethoxy]piperidine-1-carboxylate

The title compound is obtained as a yellow oil in analogy to Method B from 399.7 mg of benzyl 4-[4-(5-bromopyrimidin-2-yloxy)phenyl]-3-[4-(3-methoxypropyl)-3,4-dihydro-2H-benzo[1,4]oxazin-6-ylmethoxy]-5-triisopropylsilanyloxypiperidine-1-carboxylate.
Rf (EtOAc/heptane 2:1) = 0.24; Rt = 4.92.

### b) Benzyl 4-[4-(5-bromopyrimidin-2-yloxy)]phenyl]-3-[4-(3-methoxypropyl)-3,4-dihydro-2H-benzo[1,4]oxazin-6-ylmethoxy]-5-triisopropylsilanyloxypiperidine-1-carboxylate

The title compound is obtained as a yellow oil in analogy to Method D from 0.2 g of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1c), 0.3292 g of 5-bromo-2-chloropyrimidine and 0.2427 g of potassium carbonate. Rf (EtOAc/heptane 1:2) = 0.27.

### Example 80:

### 5-[4-(3-Methoxypropyl)-3,4-dihydro-2H-benzo[1,4]oxazin-6-ylmethoxy]-4-[4-(1-oxypyridin-2-yloxy)phenyl]piperidin-3-ol

The title compound is obtained as a colourless film in analogy to Method A from 27.1 mg of benzyl 3-hydroxy-5-[4-(3-methoxypropyl)-3,4-dihydro-2H-benzo[1,4]oxazin-6-ylmethoxy]-4-[4-(1 -oxypyridin-2-yloxy)phenyl]piperidine-1-carboxylate.

The starting materials are prepared as follows:

### a) Benzyl 3-hydroxy-5-[4-(3-methoxypropyl)-3,4-dihydro-2H-benzo[1,4]oxazin-6-ylmethoxy]-4-[4-(1-oxypyridin-2-yloxy)phenyl]piperidine-1-carboxylate

The title compound is obtained as a white foam in analogy to Method B from 38.6 mg of benzyl 3-[4-(3-methoxypropyl)-3,4-dihydro-2H-benzo[1,4]oxazin-6-ylmethoxy]-4-[4-(1-oxypyridin-2-yloxy)phenyl]-5-triisopropylsilanyloxypiperidine-1-carboxylate.
Rf (dichloromethane/methanol/25% conc. ammonia 200:20:1) = 0.27; Rt = 3.93.

### b) Benzyl 3-[4-(3-methoxypropyl)-3,4-dihydro-2H-benzo[1,4]oxazin-6-ylmethoxyl-4-[4-(1-oxypyridin-2-yloxy)phenyl]-5-triisopropylsilanyloxypiperidine-1-carboxylate

0.6 ml of a boron-tetrahydrofuran complex solution (1 M in tetrahydrofuran) is added dropwise to a solution of 134.8 mg of benzyl 3-[4-(3-methoxypropyl)-3-oxo-3,4-dihydro-2H-benzo[1,4]oxazin-6-ylmethoxy]-4-[4-(1-oxypyridin-2-yloxy)phenyl]-5-triisopropylsilanyloxy-piperidine-1-carboxylate in 2 ml of tetrahydrofuran at 0°C. After 5 hours, 2 ml of methanol are slowly added at 0°C, and the resulting mixture is evaporated. The title compound is obtained as a white film from the residue by flash chromatography (SiO₂ 60F).
Rf (dichloromethane/methanol/25% conc. ammonia 200:10:1) = 0.17.

### c) Benzyl 3-[4-(3-methoxypropyl)-3-oxo-3,4-dihydro-2H-benzo[1,4]oxazin-6-ylmethoxyl-4-[4-(1-oxypyridin-2-yloxy)phenyl]-5-triisopropylsilanyloxypiperidine-1-carboxylate

144 mg of m-chloroperbenzoic acid are added to a solution of 162.9 mg of benzyl 3-[4-(3-methoxypropyl)-3-oxo-3,4-dihydro-2H-benzo[1,4]oxazin-6-ylmethoxy]-4-[4-(pyridin-2-yloxy)-phenyl]-5-triisopropylsilanyloxypiperidine-1-carboxylate in 5 ml of dichloromethane, and the mixture is stirred at room temperature for 90 minutes. The reaction mixture is evaporated, and the title compound is obtained as a white solid from the residue by flash chromatography (SiO₂ 60F). Rf (dichloromethane/methanol/25% conc. ammonia 200:10:1) = 0.22.

### d) Benzyl 3-[4-(3-methoxypropyl)-3-oxo-3,4-dihydro-2H-benzo[1,4]oxazin-6-ylmethoxy]-4-[4-(pyridin-2-yloxy)phenyl]-5-triisopropylsilanyloxypiperidine-1-carboxylate

The title compound is obtained as a yellow oil in analogy to Method D from 0.2 g of benzyl 4-(4-hydroxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate (Example 1d), 0.19 g of 2-chloropyridine and 0.231 g of potassium carbonate. Rf (EtOAc/heptane 2:3) = 0.16.

### Example 83:

### 5-[4-(3-Methoxypropyl)-3,4-dihydro-2H-benzo[1,4]oxazin-6-ylmethoxy]-4-[4-(1-oxypyridin-3-yloxy)phenyl]piperidin-3-ol

The title compound is obtained as a colourless film in analogy to Method A from 25.4 mg of benzyl 3-hydroxy-5-[4-(3-methoxypropyl)-3,4-dihydro-2H-benzo[1,4]oxazin-6-ylmethoxy]-4-[4-(1 -oxypyridin-3-yloxy)phenyl]piperidine-1-carboxylate.

The starting materials are prepared as follows:

### a) Benzyl 3-hydroxy-5-[4-(3-methoxypropyl)-3,4-dihydro-2H-benzo[1,4]oxazin-6-ylmethoxy]-4-[4-(1 -oxypyridin-3-yloxy)phenyl]piperidine-1-carboxylate

The title compound is obtained as a white foam in analogy to Method B from 48.3 mg of benzyl 3-[4-(3-methoxypropyl)-3,4-dihydro-2H-benzo[1,4]oxazin-6-ylmethoxy]-4-[4-(1-oxypyridin-3-yloxy)phenyl]-5-triisopropylsilanyloxypiperidine-1-carboxylate.
Rf (dichloromethane/methanol/25% conc. ammonia 200:20:1) = 0.29; Rt = 4.05.

### b) Benzyl 3-[4-(3-methoxypropyl)-3,4-dihydro-2H-benzo[1,4]oxazin-6-ylmethoxy]-4-[4-(1-oxypyridin-3-yloxy)phenyl]-5-triisopropylsilanyloxypiperidine-1-carboxylate

0.95 ml of a borane-tetrahydrofuran complex solution (1 M in tetrahydrofuran) is added dropwise to a solution of 194.5 mg of benzyl 3-[4-(3-methoxypropyl)-3-oxo-3,4-dihydro-2H-benzo[1,4]oxazin-6-ylmethoxy]-4-[4-(1-oxypyridin-3-yloxy)phenyl]-5-triisopropylsilanyloxy-piperidine-1-carboxylate in 2 ml of tetrahydrofuran at 0°C. After 30 minutes, 0.9 ml of methanol is slowly added at 0°C, and the resulting mixture is concentrated. The title compound is obtained as a white foam from the residue by flash chromatography (SiO₂ 60F). Rf (dichloromethane/methanol/25% conc. ammonia 200:10:1) = 0.14.

### c) Benzyl 3-[4-(3-methoxypropyl)-3-oxo-3,4-dihydro-2H-benzo[1,4]oxazin-6-ylmethoxy]-4-[4-(1-oxypyridin-3-yloxy)phenyl]-5-triisopropylsilanyloxypiperidine-1-carboxylate

241.1 mg of m-chloroperbenzoic acid are added to a solution of 263.8 mg of benzyl 3-[4-(3-methoxypropyl)-3-oxo-3,4-dihydro-2H-benzo[1,4]oxazin-6-ylmethoxy]-4-[4-(pyridin-3-yloxy)-phenyl]-5-triisopropylsilanyloxypiperidine-1-carboxylate in 3.3 ml of dichloromethane and stirred at room temperature for 15 hours. The reaction mixture is concentrated, and the title compound is obtained as a white foam from the residue by flash chromatography (SiO₂ 60F). Rf (dichloromethane/methanol/25% conc. ammonia 200:10:1) = 0.17.

### d) Benzyl 3-[4-(3-methoxypropyl)-3-oxo-3,4-dihydro-2H-benzo[1,4]oxazin-6-ylmethoxy]-4-[4-(pyridin-3-yloxy)phenyl]-5-triisopropylsilanyloxypiperidine-1-carboxylate

The title compound is obtained as a yellow oil in analogy to Method C from 0.81 g of benzyl 4-(4-hydroxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate (Example 1d), 0.5601 g of 3-pyridine boronic acid, 0.4506 g of copper(II) acetate and 0.77 ml of triethylamine.
Rf (EtOAc/heptane 1:1) = 0.10.

### Example 84:

### 5-[4-(3-Methoxypropyl)-3,4-dihydro-2H-benzo[1,4]oxazin-6-ylmethoxy]-4-[4-(1-oxypyridin-4-yloxy)phenyl]piperidin-3-ol

The title compound is obtained as a colourless film in analogy to Method A from 150 mg of benzyl 3-hydroxy-5-[4-(3-methoxypropyl)-3,4-dihydro-2H-benzo[1,4]oxazin-6-ylmethoxy]-4-[4-(1 -oxypyridin-4-yloxy)phenyl]piperidine-1-carboxylate.

The starting materials are prepared as follows:

### a) Benzyl 3-hydroxy-5-[4-(3-methoxypropyl)-3,4-dihydro-2H-benzo[1,4]oxazin-6-ylmethoxy]-4-[4-(1-oxypyridin-4-yloxy)phenyl]piperidine-1-carboxylate

The title compound is obtained as a brownish resin in analogy to Method B from 171 mg of benzyl 3-[4-(3-methoxypropyl)-3,4-dihydro-2H-benzo[1,4]oxazin-6-ylmethoxy]-4-[4-(1-oxy-pyridin-4-yloxy)phenyl]-5-triisopropylsilanyloxypiperidine-1-carboxylate. Rf (CH₂Cl₂/MeOH 95:5) = 0.28; Rt = 4.06.

### b) Benzyl 3-[4-(3-methoxypropyl)-3,4-dihydro-2H-benzo[1,4]oxazin-6-ylmethoxy]-4-[4-(1-oxypyridin-4-yloxy)phenyl]-5-triisopropylsilanyloxypiperidine-1-carboxylate

The title compound is obtained as a brown resin in analogy to Method D from 400 mg of benzyl 4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 1 c), 91.9 mg of 4-chloropyridine N-oxide and 156.8 mg of potassium carbonate.
Rf (CH₂Cl₂/MeOH 95:5) = 0.38.

### Example 85:

### (2-{5-[4-(3-Methoxypropyl)-3,4-dihydro-2H-benzo[1,4]oxazin-6-ylmethoxy]-4-[4-(4-methyl-3,4-dihydro-2H-benzo[1,4]oxazin-7-yloxy)phenyl]piperidin-3-yloxy}ethyl)methylamine

246 mg of sodium/mercury amalgam are added to a solution of 110 mg of N-{2-[5-[4-(3-methoxypropyl)-3,4-dihydro-2H-benzo[1,4]oxazin-6-ylmethoxy]-4-[4-(4-methyl-3,4-dihydro-2H-benzo[1,4]oxazin-7-yloxy)phenyl]-1-(toluene-4-sulphonyl)piperidin-3-yloxy]ethyl}-4,N-dimethylbenzene sulphonamide and 80 mg of anhydrous sodium dihydrogenphosphate in 4 ml of methanol under argon. After the reaction is complete, the reaction mixture is diluted with water and extracted with ethyl acetate (3x) - the combined organic phases are washed with brine, dried with sodium sulphate and evaporated. The title compound is obtained as a white resin from the residue by flash chromatography (SiO₂ 60F).

The starting materials are prepared as follows:

### a) N-{2-[5-[4-(3-Methoxypropyl)-3,4-dihydro-2H-benzo[1,4]oxazin-6-ylmethoxy]-4-[4-(4-methyl-3,4-dihydro-2H-benzo[1,4]oxazin-7-yloxy)phenyl]-1-(toluene-4-sulphonyl)-piperidin-3-yloxy]ethyl}-4,N-dimethylbenzene sulphonamide

95 mg of sodium hydride dispersion (60%) are added to a solution of 145 mg of 5-[4-(3-methoxypropyl)-3,4-dihydro-2H-benzo[1,4]oxazin-6-ylmethoxy]-4-[4-(4-methyl-3,4-dihydro-2H-benzo[1,4]oxazin-7-yloxy)phenyl]-1-(toluene-4-sulphonyl)piperidin-3-ol and 740 mg of 2-[methyl(toluene-4-sulphonyl)amino]ethyl toluene-4-sulphonate in 5 ml of tetrahydrofuran while stirring at room temperature. The reaction mixture is stirred at 45°C for 72 hours. The mixture is poured into 1 M aqueous sodium bicarbonate solution (5 ml) and extracted with tert-butyl methyl ether (2 x 50 ml). The organic phases are washed successively with water (2 x 10 ml) and brine (1 x 10 ml), dried with sodium sulphate and evaporated. The title compound is obtained from the residue by flash chromatography (SiO₂ 60F). Rf = 0.08 (EtOAc/heptane 1:1); Rt = 5.83.

### b) 5-[4-(3-Methoxypropyl)-3,4-dihydro-2H-benzo[1,4]oxazin-6-ylmethoxy]-4-[4-(4-methyl-3,4-dihydro-2H-benzo[1,4]oxazin-7-yloxy)phenyl]-1-(toluene-4-sulphonyl)piperidin-3-ol

A mixture of 105.7 mg of 5-[4-(3-methoxypropyl)-3,4-dihydro-2H-benzo[1,4]oxazin-6-ylmethoxy]-4-[4-(4-methyl-3,4-dihydro-2H-benzo[1,4]oxazin-7-yloxy)phenyl]piperidin-3-ol (Example 58) in 3.3 ml of ethyl acetate and 3.3 ml of 2N aqueous sodium carbonate solution is mixed with 37.7 mg of p-toluenesulphonyl chloride and stirred for 15 hours. The phases are separated, and the organic phase is extracted twice more with 10 ml of ethyl acetate each time. The combined organic phases are washed once with brine (10 ml), dried with sodium sulphate and concentrated. The title compound remains as a white foam. Rt = 5.05.

### c) 2-[Methyl(toluene-4-sulphonyl)amino]ethyl toluene-4-sulphonate

10.55 g of p-toluenesulphonyl chloride are added to a solution of 2.13 ml of 2-(methylamino)-ethanol and 8 ml of triethylamine in 25 ml of dichloromethane while cooling in ice, and the mixture is stirred at room temperature for 20 hours. The reaction mixture is diluted with 100 ml of tert-butyl methyl ether and washed with 0.1 M HCl (50 ml), water (50 ml) and brine (50 ml). The organic phase is dried with sodium sulphate and evaporated, whereupon the title compound crystallizes as white solid. Rt = 4.67.

## Claims

1. Use of a compound of formula 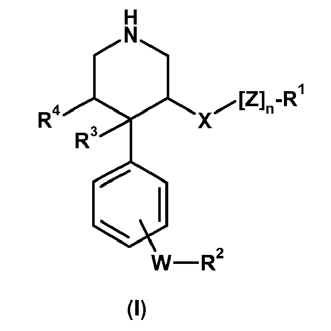 in which
(A) R¹ is aryl when R² is tetrazolyl or imidazolyl, each of which may be substituted by C₁₋₆alkoxy-C₁₋₆alkoxy-C₁₋₆alkyl, C₁₋₆alkoxy-C₁₋₆alkyl, aryloxy-C₁₋₆alkyl, heterocyclyloxy-C₁₋₆alkyl; or
(B) R¹ is aryl when X is -O-CH-R⁵-CONR⁶-; or
(C) R¹ is aryl when Z is -alk-NR⁶-, where alk is C₁₋₆alkylene, and n is 1; or
(D) R¹ is aryl which is substituted by 1-4 acetamidinyl-C₁₋₆alkyl, acyl-C₁₋₆alkoxy-C₁₋₆alkyl, (N-acyl)-C₁₋₆alkoxy-C₁₋₆alkylamino, C₁₋₆alkoxy, C₁₋₆alkoxy-C₁₋₆alkoxy, C₁₋₆alkoxy-C₁₋₆alkoxy-C₁₋₆alkyl, C₁₋₆alkoxy-C₁₋₆alkyl, (N-C₁₋₆alkoxy)-C₁₋₆alkylaminocarbonyl-C₁₋₆alkoxy, (N-C₁₋₆alkoxy)-C₁₋₆alkylaminocarbonyl-C₁₋₆alkyl, C₁₋₆alkoxy-C₁₋₆alkylcarbamoyl, C₁₋₆alkoxy-C₁₋₆alkylcarbonyl, C₁₋₆alkoxy-C₁₋₆alkylcarbonylamino, 1-C₁₋₆alkoxy-C₁₋₆alkylimidazol-2-yl, 2-C₁₋₆alkoxy-C₁₋₆alkyl-4-oxoimidazol-1-yl, 1-C₁₋₆alkoxy-C₁₋₆alkyltetrazol-5-yl, 5-C₁₋₆alkoxy-C₁₋₆alkyltetrazol-1-yl, 6-alkoxyamino-carbonyl-C₁₋₆alkoxy, C₁₋₆alkoxyaminocarbonyl-C₁₋₆alkyl, C₁₋₆alkoxycarbonyl, C₁₋₆alkoxycarbonyl-C₁₋₆alkoxy, C₁₋₆alkoxycarbonyl-C₁₋₆alkyl, C₁₋₆alkoxycarbonylamino-C₁₋₆alkoxy, C₁₋₆alkoxycarbonylamino-C₁₋₆alkyl, C₁₋₆alkyl, (N-C₁₋₆alkyl)-C₁₋₆alkoxy-C₁₋₆alkylcarbamoyl, (N-C₁₋₆alkyl)-C₁₋₆alkoxy-C₁₋₆alkylcarbonylamino, (N-C₁₋₆alkyl)-C₁₋₆alkoxy-carbonylamino, (N-C₁₋₆-alkyl)-C₀₋₆alkylcarbonylamino-C₁₋₆alkoxy, (N-C₁₋₆alkyl)-C₀₋₆alkylcarbonylamino-C₁₋₆alkyl, (N-C₁₋₆alkyl)-C₁₋₆alkylsulphonylamino-C₁₋₆alkoxy, (N-C₁₋₆alkyl)-C₁₋₆alkylsulphonylamino-C₁₋₆alkyl, C₁₋₆alkylamidinyl, C₁₋₆alkylaminocarbonyl-C₁₋₆alkoxy, di-C₁₋₆alkylaminocarbonyl-C₁₋₆alkoxy, C₁₋₆alkylaminocarbonyl-C₁₋₆alkoxy-C₁₋₆alkyl, C₁₋₆alkylaminocarbonyl-C₁₋₆alkyl, C₁₋₆alkylamino-carbonylamino-C₁₋₆alkoxy, C₁₋₆alkylaminocarbonylamino-C₁₋₆alkyl, di-C₁₋₆alkylaminocarbonyl-C₁₋₆alkyl, C₁₋₆alkylamino-C₂₋₆alkoxy, di-C₁₋₆alkylamino-C₂₋₆alkoxy, C₁₋₆alkylamino-C₁₋₆alkyl, di-C₁₋₆alkylamino-C₁₋₆alkyl, C₁₋₆alkylcarbamoyl, di-C₁₋₆alkylcarbamoyl, C₀₋₆alkylcarbonylamino-C₁₋₆alkoxy, C₀₋₆alkylcarbonylamino, C₀₋₆alkylcarbonylamino-C₁₋₆alkyl, C₁₋₆alkylcarbonyloxy-C₁₋₆alkoxy, C₁₋₆alkylcarbonyloxy-C₁₋₆alkyl, C₁₋₆alkylsulphonyl, C₁₋₆alkylsulphonyl-C₁₋₆alkoxy, C₁₋₆alkylsulphonyl-C₁₋₆alkyl, C₁₋₆alkylsulphonylamino-C₁₋₆alkoxy, C₁₋₆alkylsulphonylamino-C₁₋₆alkyl, carbamoyl, carbamoyl-C₁₋₆alkoxy, carbamoyl-C₁₋₆alkyl, carboxy-C₁₋₆alkoxy, carboxy-C₁₋₆alkoxy-C₁₋₆alkyl, carboxy-C₁₋₆alkyl, cyano, cyano-C₁₋₆alkoxy, cyano-C₁₋₆alkyl, C₃₋₆cycloalkylcarbonyl-amino-C₁₋₆alkoxy, C₃₋₆cycloalkylcarbonylamino-C₁₋₆alkyl, cyclopropyl-C₁₋₆alkyl, O,N-dimethylhydroxylamino-C₁₋₆alkyl, halogen, hydroxy-C₂₋₆alkoxy-C₁₋₆alkoxy, hydroxy-C₂₋₆alkoxy-C₁₋₆alkyl, hydroxy-C₁₋₆alkyl, (N-hydroxy)-C₁₋₆alkylaminocarbonyl-C₁₋₆alkoxy, (N-hydroxy)-C₁₋₆alkylaminocarbonyl-C₁₋₆alkyl, (N-hydroxy)aminocarbonyl-C₁₋₆alkoxy, (N-hydroxy)aminocarbonyl-C₁₋₆alkyl, 2-oxooxazolidinyl-C₁₋₆alkoxy, 2-oxooxazolidinyl-C₁₋₆alkyl, O-methyloximyl-C₁₋₆alkyl or trifluoromethyl; or
(E) R¹ is aryl which is substituted by 3-acetamidomethylpyrrolidinyl, 3-C₁₋₆alkoxy-C₁₋₆alkyl-pyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolylalkoxy, imidazolylalkyl, 2-methylimidazolylalkoxy, 2-methylimidazolylalkyl, 3-methyl-[1,2,4]-oxadiazol-5-ylalkoxy, 5-methyl-[1,2,4]-oxadiazol-3-ylalkoxy, 3-methyl-[1,2,4]-oxadiazol-5-ylalkyl, 5-methyl-[1,2,4]-oxadiazol-3-ylalkyl, 4-methylpiperazinyl, 5-methyltetrazol-1-ylalkoxy, 5-methyltetrazol-1-ylalkyl, morpholinyl, [1,2,4]-oxadiazol-5-ylalkoxy, [1,2,4]-oxadiazol-5-ylalkyl, oxazol-4-ylalkoxy, oxazol-4-ylalkyl, 2-oxo-[1,3]oxazinyl, 2-oxooxazolidinyl, 2-oxoimidazolidinyl, 2-oxopyrrolidinyl, 4-oxopiperidinyl, 2-oxopyrrolidinylalkoxy, 2-oxopyrrolidinylalkyl, 2-oxotetrahydropyrimidinyl, 4-oxothiomorpholinyl, piperazinyl, piperidinyl, pyrrolidinyl, pyrrolyl, [1,2,4]-triazol-1-ylalkoxy, [1,2,4]-triazol-4-ylalkoxy, [1,2,4]-triazol-1-ylalkyl, [1,2,4]-triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5-ylalkyl, thiazol-4-ylalkoxy, thiazol-4-ylalkyl, thiomorpholinyl; or
(F) R¹ is heterocyclyl optionally substituted as indicated under (D) or (E), in particular benzo[1,3]dioxolyl, benzofuranyl, benzooxazolyl, dihydrobenzofuranyl, 3,4-dihydro-2H-benzo[1,4]oxazinyl, dihydro-3H-benzo[1,4]oxazinyl, dihydro-2H-benzo[1,4]thiazinyl, 2,3-dihydroindolyl, dihydro-1H-pyrido[2,3-b][1,4]oxazinyl, 1,1-dioxodihydro-2H-benzo[1,4]thiazinyl, indazolyl, indolyl, [1,5]naphthyridyl, oxazolyl, 2-oxoazepanyl, 3-oxo-4H-benzo[1,4]oxazinyl, 2-oxobenzooxazolyl, 3-oxo-4H-benzo[1,4]thiazinyl, 2-oxodihydrobenzo[e][1,4]diazepinyl, 2-oxodihydrobenzo-[d][1,3]oxazinyl, 2-oxodihydro-1H-quinazolinyl, 4-oxodihydroimidazolyl, 2-oxo-1,3-dihydroindolyl, 1-oxo-3H-isobenzofuranyl, 2-oxopiperidinyl, 2-oxo-1 H-pyrido[2,3-b][1,4]oxazinyl, 1-oxopyridyl, 2-oxotetrahydrobenzo[e][1,4]diazepinyl, 4-oxo-3H-thieno[2,3-d]pyrimidinyl, 5-oxo-4H-[1,2,4]triazinyl, phthalazinyl, pyrazolyl, 1H-pyrrolizinyl, 1 H-pyrrolo[2,3-b]pyridyl, pyrrolyl, tetrahydroquinoxalinyl, tetrahydropyranyl, triazinyl or 1,1,3-trioxodihydro-2H-1lambda*6*-benzo[1,4]thiazinyl;
R² is phenyl or a heterocyclyl which is linked via a C atom, each of which radicals may be substituted by 1-4 C₁₋₆alkanoyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylenedioxy, C₁₋₆alkylsulphanyl, C₁₋₆alkylsulphonyl, optionally N-mono- or N,N-di-C₁₋₆-alkylated amino, optionally N-mono- or N,N-di-C₁₋₆-alkylated carbamoyl, optionally esterified carboxy, cyano, cyano-C₁₋₆alkyl, C₃₋₈cycloalkoxy, C₃₋₈cydoalkyl, halogen, hydroxy-C₁₋₆alkyl, nitro, oxide, oxo, trifluoromethyl, trifluoromethoxy or optionally N-C₁₋₆-alkylated piperazinyl-C₁₋₆alkyl;
R³ is hydrogen, hydroxy, C₁₋₆alkoxy or C₂₋₆alkenyloxy;
R⁴ is C₁₋₆alkoxy, C₁₋₆alkoxy-C₁₋₆alkoxy, optionally N-mono- or N,N-di-C₁₋₆-alkylated amino-C₁₋₆alkoxy, optionally N-C₁₋₆-alkylated C₁₋₆alkoxycarbonylamino-C₁₋₆alkoxy, optionally N-C₁₋₆-alkylated C₁₋₆alkylcarbonylamino-C₁₋₆alkoxy, C₃₋₈cycloalkyloxy, C₃₋₈cycloalkyloxy-C₁₋₆alkoxy, hydroxy, hydroxy-C₂₋₆alkoxy, hydroxy-C₂₋₆alkoxy-C₁₋₆alkoxy, heterocyclyl-C₁₋₆alkoxy, heterocyclyloxy, heterocyclyloxy-C₁₋₆alkoxy or oxo;
R⁵ is acyl, C₂₋₈alkenyl, C₁₋₆alkyl, aryl-C₁₋₆alkyl or hydrogen;
R⁶ is acyl, C₁₋₆alkoxy-C₁₋₆alkyl, C₁₋₆alkyl or aryl-C₁₋₆alkyl or hydrogen;
R⁷ is C₁₋₆alkoxycarbonyl-C₁₋₆alkyl, C₁₋₆alkyl, carboxy-C₁₋₆alkyl or hydrogen;
W is oxygen, methylene or difluoromethylene;
X is a bond, oxygen or sulphur, where the bond originating from an oxygen or sulphur atom leads to a saturated C atom of group Z or to R¹, or a group >CH-R⁵, >CHOR⁶, -O-CO-, >CO, >C=NOR⁷, -O-CHR⁵- or -O-CHR⁵-CO-NR⁶-;
Z is C₁₋₆alkylene, C₂₋₈alkenylene, hydroxy-C₁₋₆alkylidene, -O-, -S-, -O-alk-, -S-alk-, -alk-O-, -alk-S- or-alk-NR⁶-, where alk is C₁₋₆alkylene; and where
(a) if Z is -O-alk- or -S-alk-, then X is -CHR⁵-; and
(b) if X is a bond, then Z is C₂₋₈alkenylene, -alk-O- or -alk-S-;
n is 1 or, if X is -O-CO- or -O-CHR⁵-CO-NR⁶-, 0 or 1;
or pharmaceutically acceptable salt or prodrug thereof, or where one or more atoms are replaced by their stable, non-radioactive isotopes;
for the manufacture of a medicament for the inhibition of beta-secretase, cathepsin D, plasmepsin II and/or HIV-protease.

2. Use according to claim 1 of a compound of formula (IA) 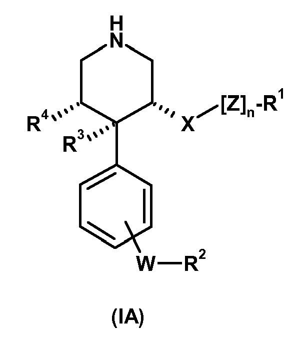 where R¹, R², R³, R⁴, W, X, Z and n are each as defined in claim 1.

3. Use according to any one of claims 1 to 2, where,
R¹ is heterocyclyl, substituted as stated under (D) or (E) in Claim 1, where heterocyclyl is selected from benzo[1,3]dioxolyl, benzofuranyl, benzooxazolyl, dihydrobenzofuranyl, 3,4-dihydro-2H-benzo[1,4]oxazinyl, dihydro-3H-benzo[1,4]oxazinyl, dihydro-2H-benzo[1,4]thiazinyl, 2,3-dihydroindolyl, dihydro-1H-pyrido[2,3-b][1,4]oxazinyl, 1,1-dioxo-dihydro-2H-benzo[1,4]thiazinyl, indazolyl, indolyl, [1,5]naphthyridyl, oxazolyl, 2-oxoazepanyl, 3-oxo-4H-benzo[1,4]oxazinyl, 2-oxobenzooxazolyl, 3-oxo-4H-benzo[1,4]thiazinyl, 2-oxo-dihydrobenzo[e][1,4]diazepinyl, 2-oxodihydrobenzo[d][1,3]oxazinyl, 2-oxodihydro-1 H-quinazolinyl, 4-oxodihydroimidazolyl, 2-oxo-1,3-dihydroindolyl, 1-oxo-3H-isobenzofuranyl, 2-oxopiperidinyl, 2-oxo-1 H-pyrido[2,3-b][1,4]oxazinyl, 1-oxopyridyl, 2-oxotetrahydro-benzo[e][1,4]diazepinyl, 4-oxo-3H-thieno[2,3-d]pyrimidinyl, 5-oxo-4H-[1,2,4]triazinyl, phthalazinyl, pyrazolyl, 1 H-pyrrolizinyl, 1 H-pyrrolo[2,3-b]pyridyl, pyrrolyl, tetrahydroquinoxalinyl, tetrahydropyranyl, triazinyl and 1,1,3-trioxodihydro-2H-1lambda*6*-benzo[1,4]thiazinyl.

4. Use according to any one of claims 1 to 3, where,
where X is oxygen, sulphur, -O-CHR⁵-, -O-CHR⁵-CO-NR⁶- or-CO- and Z is methylene or -alk-O-.

5. Use according to any one of claims 1 to 4, where,
R² in the meaning of a heterocyclyl linked via a C atom is a radical selected from pyridyl, pyrimidinyl, pyrazinyl, benzo[b]thienyl, quinolyl, isoquinolyl, quinoxalinyl, indolyl, 3,4-dihydro-2H-benzo[1,4]oxazinyl, 2,3-dihydrobenzofuranyl and benzofuranyl.

6. Use of a compound of the general formula (I) or (IA), or a pharmaceutically acceptable salt thereof, according to any one of claims 1 to 5 for the preparation of a medication for the prevention, delay of progression or treatment of Alzheimer Disease, malaria or HIV infection.

7. Pharmaceutical preparation for the prevention, delay of progression or treatment of Alzheimer disease, malaria or HIV infection containing a compound of the general formula (I) or (IA), or a pharmaceutically acceptable salt thereof, according to any one of claims 1 to 5 as well as commonly used ingredients.
